# FASCICULE DE BREVET EUROPEEN

(11) **EP 1 456 160 B1**
(45) Date de publication et mention de la délivrance du brevet: **20.01.2010**
(21) Numéro de dépôt: 02804598.7
(22) Date de dépôt: 06.12.2002
(51) Int. Cl.: C07C 43/23, A61K 31/085, A61K 31/045, A61K 31/135, C07C 33/26, C07C 215/68, A61P 17/00

(54) **ANALOGUES DE LA VITAMINE D**
VITAMIN-D-ANALOGA
VITAMIN D ANALOGUES

(30) Priorité: 10.12.2001 FR 0115924
(43) Date de publication de la demande: 15.09.2004
(73) Titulaire: GALDERMA RESEARCH & DEVELOPMENT, 06410 Biot (FR)
(72) Inventeur: BERNARDON, Jean-Michel, F-06000 Nice (FR); BIADATTI, Thibaud, F-06650 Opio (FR)
(74) Mandataire: Allab, Myriam
(86) Numéro de dépôt international: PCT/FR2002/004216
(87) Numéro de publication internationale: WO 2003/050067

(56) Documents cités:
- WO-A-01/38303

## Description

L'invention se rapporte, à titre de produits industriels nouveaux et utiles, à des composés tri-aromatiques, analogues de la vitamine D.

L'invention se rapporte également à leur procédé de préparation et à leur utilisation dans des compositions pharmaceutiques destinées à un usage en médecine humaine ou vétérinaire, ou bien encore dans des compositions cosmétiques.

Les composés selon l'invention ont une activité marquée dans les domaines de la prolifération et de la différenciation cellulaire et trouvent des applications plus particulièrement dans le traitement topique et systémique des affections dermatologiques (ou autres) liées à un désordre de la kératinisation, des affections à composante inflammatoire et/ou immunoallergique et de l'hyperprolifération des tissus d'origine ectodermique (peau, épithélium...), qu'elle soit bénigne ou maligne. Ces composés peuvent en outre être utilisés pour lutter contre le vieillissement de la peau, qu'il soit photoinduit ou chronologique et traiter les troubles de la cicatrisation.

On peut également utiliser les composés selon l'invention dans des compositions cosmétiques pour l'hygiène corporelle et capillaire.

La vitamine D est une vitamine essentielle pour la prévention et le traitement des défauts de minéralisation du cartilage (rachitisme), et de l'os (ostéomalacie), et même de certaines formes d'ostéoporose chez le sujet âgé. Mais il est maintenant admis que ses fonctions s'étendent bien au delà de la régulation du métabolisme osseux et de l'homéostasie calcique. Parmi celles-ci, peuvent être citées ses actions sur la prolifération et sur la différenciation cellulaire et le contrôle des défenses immunitaires. Leur découverte a ouvert la voie à de nouvelles approches thérapeutiques en dermatologie, cancérologie, ainsi que dans le domaine des maladies auto-immunes et celui des transplantations d'organes ou de tissus.

Un apport thérapeutique efficace s'est longtemps heurté à la toxicité de cette vitamine (hypercalcémie parfois mortelle). Actuellement, des analogues structuraux de la vitamine D sont synthétisés, dont certains ne conservent que les propriétés différenciatrices et n'ont pas d'action sur le métabolisme calcique. La Demanderesse a déjà proposé dans la demande WO 01/38303 de nouveaux composés analogues de la vitamine D qui montrent une activité sélective sur la prolifération et sur la différenciation cellulaire sans présenter de caractère hypercalcémiant.

La Demanderesse vient de découvrir, de manière surprenante, que certains composés non spécifiquement décrits dans la demande WO 01/38303 montrent une activité biologique très supérieure à celle des composés spécifiquement décrits. Cette activité est si forte qu'elle est supérieure ou égale à l'activité de la 1,25-dihydroxyvitamine D₃.

Ainsi, la présente invention concerne des composés, caractérisés par le fait qu'ils sont pris, seuls ou en mélanges, dans le groupe constitué par :
1-{5-[4'-(1-Ethyl-1-hydroxypropyl)-6-methyl-2'-propylbiphenyl-3-yloxymethyl]-2-hydroxymethylphenyl}methanol;
2-{5-[6,2'-Diethyl-4'-(1-ethyl-1-hydroxypropyl)biphenyl-3-yloxymethyl]-2-hydroxymethyl-phenyl}methanol;
3-{4-[6-Ethyl-4'-(1-ethyl-1-hydroxypropyl)-2'-propylbiphenyl-3-yloxymethyl]-2-hydroxymethylphenyl}methanol;
4-{4-[6-Ethyl-4'-(1-ethyl-1-hydroxypropyl)-2'-isopropylbiphenyl-3-yloxymethyl]-2-hydroxymethylphenyl}methanol;
5-(4-{2-[4'-(1-Ethyl-1-hydroxypropyl)-6-methyl-2'-propylbiphenyl-3-yl]ethyl}-2-hydroxymethylphenyl)methanol;
6-{4-[4'-(1-Ethyl-1-hydroxypropyl)-6-methyl-2'-propylbiphenyl-3-ylmethoxy]-2-hydroxymethylphenyl}methanol;
7-(4-{[4'-(1-Ethyl-1-hydroxypropyl)-6-methyl-2'-propylbiphenyl-3-ylamino]methyl}-2-hydroxymethylphenyl)methanol;
8-[4-({[4'-(1-Ethyl-1-hydroxypropyl)-6-methyl-2'-propylbiphenyl-3-yl]methylamino}methyl)-2-hydroxymethylphenyl]methanol;
9-[4-({Ethyl-[4'-(1-ethyl-1-hydroxypropyl)-6-methyl-2'-propylbiphenyl-3-yl]amino}methyl)-2-hydroxymethylphenyl]methanol;
10-4-({[4'-(1-Ethyl-1-hydroxypropyl)-6-methyl-2'-propylbiphenyl-3-yl]propylamino}methyl)-2-hydroxymethylphenyl]methanol;
11-(2-Hydroxymethyl-4-{2-[6-methyl-2'-propyl-4'-(2,2,2-trifluoro-1-hydroxy-1-trifluoromethyl-ethyl)biphenyl-3-yl]ethyl}phenyl)methanol;
12-{2-Hydroxymethyl-4-[6-methyl-2'-propyl-4'-(2,2,2-trifluoro-1-hydroxy-1-trifluoromethyl-ethyl)biphenyl-3-yloxymethyl]phenyl}methanol;
13-{2-Hydroxymethyl-4-[6-methyl-2'-propyl-4'-(2,2,2-trifluoro-1-hydroxy-1-trifluoromethyl-ethyl)biphenyl-3-ylmethoxylphenyl}methanol;
14-(2-Hydroxymethyl-4-{[6-methyl-2'-propyl-4'-(2,2,2-trifluoro-1-hydroxy-1-trifluoromethyl-ethyl)biphenyl-3-ylamino]methyl}phenyl)methanol;
15-[2-Hydroxymethyl-4-({N-methyl[6-methyl-2'-propyl-4'-(2,2,2-trifluoro-1-hydroxy-1-trifluoromethyl-ethyl)biphenyl-3-yl]amino}methyl)phenyl]methanol;
16-[4-({N-Ethyl[6-methyl-2'-propyl-4'-(2,2,2-trifluoro-1-hydroxy-1-trifluoromethylethyl)biphenyl-3-yl]amino}methyl)-2-hydroxymethylphenyl]methanol;
17-[2-Hydroxymethyl-4-({[6-methyl-2'-propyl-4'- (2,2,2-trifluoro-1-hydroxy-1-trifluoromethyl-ethyl)biphenyl-3-yl]N-propyl-amino}methyl)phenyl]methanol;
18-(4-{2-[6-Ethyl-4'-(1-ethyl-1-hydroxypropyl)-2'propylbiphenyl-3-yl]ethyl}-2-hydroxymethylphenyl)methanol;
19-{4-[6-Ethyl-4'-(1-ethyl-1-hydroxypropyl)-2'-propylbiphenyl-3-ylmethoxy]-2-hydroxymethylphenyl}methanol;
20-(4-{[6-Ethyl-4'-(1-ethyl-1-hydroxypropyl)-2'-propylbiphenyl-3-ylamino]methy}-2-hydroxymethylphenyl)methanol;
21-[4-({[6-Ethyl-4'-(1-ethyl-1-hydroxypropyl)-2'-propylbiphenyl-3-yl]methylamino}methyl)-2-hydroxymethylphenyl]methanol;
22-[4-({Ethyl-[6-ethyl-4'-(1-ethyl-1-hydroxypropyl)-2'-propylbiphenyl-3-yl]amino}methyl)-2-hydroxymethylphenyl]methanol;
23-[4-({[6-Ethyl-4'-(1-ethyl-1-hydroxypropyl)-2'-propylbiphenyl-3-yl]propylamino}methyl)-2-hydroxymethylphenyl]methanol;
24-(4-{2-[6-Ethyl-2'-propyl-4'-(2,2,2-trifluoro-1-hydroxy-1-trifluoromethylethyl)biphenyl-3-yl]ethyl}-2-hydroxymethylphenyl)methanol;
25-{4-[6-Ethyl-2'-propyl-4'-(2,2,2-trifluoro-1-hydroxy-1-trifluoromethyl-ethyl)biphenyl-3-yloxymethyl]-2-hydroxymethylphenyl}methanol;
26-{4-[6-Ethyl-2'-propyl-4'-(2,2,2-trifluoro-1-hydroxy-1-trifluoromethyl-ethyl)biphenyl-3-ylmethoxy]-2-hydroxymethylphenyl}methanol;
27-(4-{[6-Ethyl-2'-propyl-4'-(2,2,2-trifluoro-1-hydroxy-1-trifluoromethyl-ethyl)biphenyl-3-ylamino]methyl}-2-hydroxymethylphenyl)methanol;
28-[4-({[6-Ethyl-2'-propyl-4'-(2,2,2-trifluoro-1-hydroxy-1-trifluoromethyl-ethyl)biphenyl-3-yl]methylamino}methyl)-2-hydroxymethylphenyl]methanol;
29-[4-({N-Ethyl[6-ethyl-2'=propyl-4'-(2,2,2-trifluoro-1-hydroxy-1-trifluoromethylethyl)biphenyl-3-yl]amino}methyl)-2-hydroxymethylphenyl]methanol;
30-[4-({[6-Ethyl-2'-propyl-4'-(2,2,2-trifluoro-1-hydroxy-1-trifluoromethyl-ethyl)biphenyl-3-yl]-N-propyl-amino}methyl)-2-hydroxymethylphenyl]methanol;
31-(4-{[4'-(1-Ethyl-1-hydroxypropyl)-6,2'-dimethylbiphenyl-3-ylamino]methyl}-2-hydroxymethylphenyl)methanol.

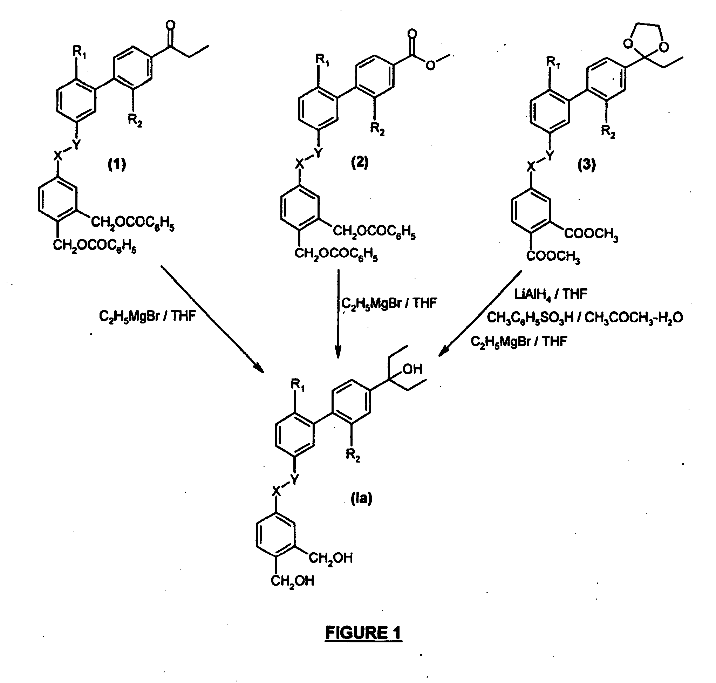

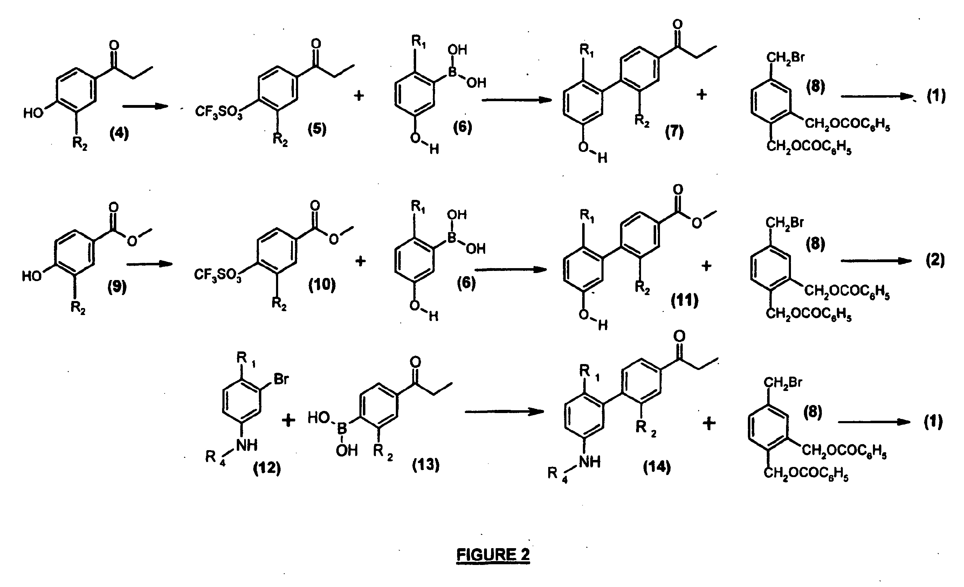

Les composés de la présente invention sont couverts par la formule générale (I) telle que décrite ci-dessous, et peuvent être préparés selon les schémas réactionnels présentés en figure 1. 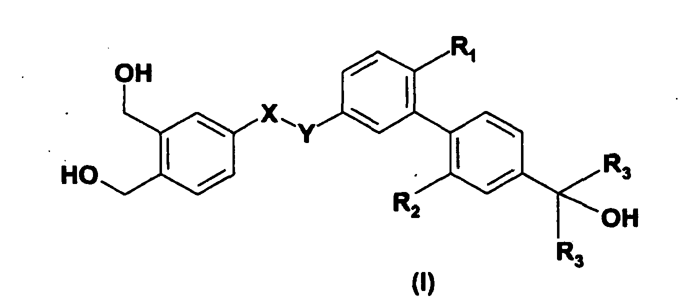 dans laquelle:
- X-Y représente les liaisons de structures suivantes:
   -CH₂-CH₂-
   -CH₂-O-
   -O-CH₂-
   -CH₂-N(R₄)-
   R₄ ayant les significations données ci-apres,
- R₁ représente un radical méthyle ou un radical éthyle,
- R₂ représente un radical éthyle, un radical propyle ou un radical isopropyle,
- R₃ représente un radical éthyle ou un radical trifluorométhyle,
- R₄ représente un atome d'hydrogène, un radical méthyle, un radical éthyle ou un radical propyle.
Lorsque -X-Y- représente une liaison de structure -CH₂-O- ou -CH₂-N(R₄)-, les composés de formule (Ia) sont préparés de préférence à partir des composés (1) et (2), obtenus selon les schémas réactionnels de la figure 2.
Les composés (1) (dans le cas où Y = O) et (2) peuvent être obtenus respectivement à partir des composés (4) et (9), tout d'abord par formation des dérivés trifluorométhane sulfonate (respectivement (5) et (10)) en présence d'anhydride trifluorométhanesulfonique et d'une base telle la triéthylamine dans le dichlorométhane. Puis par une réaction de couplage de type Suzuki avec les dérivés acide boronique (6) en présence d'un catalyseur tel le tétrakis(triphénylphosphine)palladium(0), on obtient respectivement les dérivés (7) et (11) et ensuite réaction de couplage avec les dérivés bromé (8) en présence de carbonate de potassium dans la méthyléthylcétone. Dans le cas où Y = NR₄, le composé (1) peut être obtenu à partir du composé (12), tout d'abord par une réaction de couplage de type Suzuki avec le dérivé acide boronique (13) en présence d'un catalyseur tel le tétrakis(triphénylphosphine)palladium(0), on obtient le dérivé (14) et ensuite par réaction de couplage avec les dérivés bromé (8) en présence d'hydrure de sodium dans le diméthylformamide.

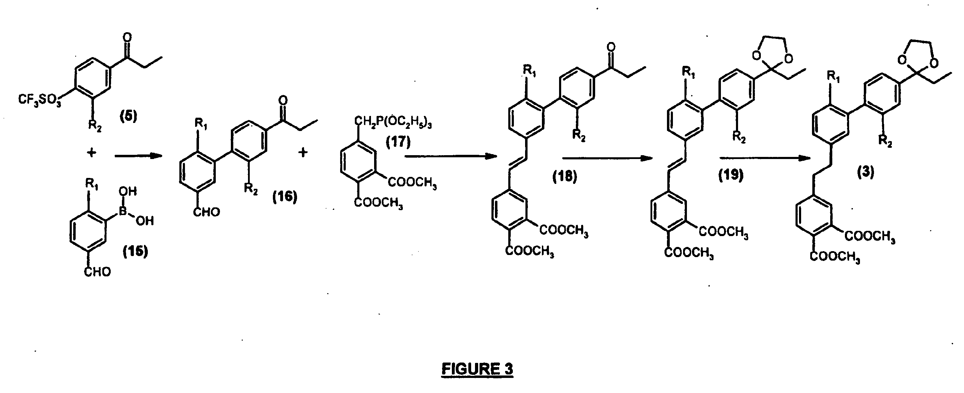

Lorsque -X-Y- représente une liaison de structure -CH₂CH₂-, les composés de formule (la) présentés dans la figure 1 sont préparés à partir des composés (3), obtenus selon le schéma réactionnel présenté en figure 3.
Par réaction de type Suzuki des dérivés trifluorométhanesulfonate (5) avec les dérivés acide boronique (15) en présence d'un catalyseur tel le tétrakis(triphénylphosphine)palladium(0) d'une base tel le carbonate de potassium dans un solvant tel le 1,2-diméthoxyéthane, on obtient les composés (16). Ces derniers sont transformés en composés (18) par une réaction de type Homer-Emmons avec le composé (17) puis en composés (19) par protection de la fonction cétonique sous forme de dioxolane (éthylène glycol, acide paratoluènesulfonique, toluène). Par réduction de la double liaison en présence de palladium sur charbon dans un solvant tel le méthanol, on obtient les composés (3).

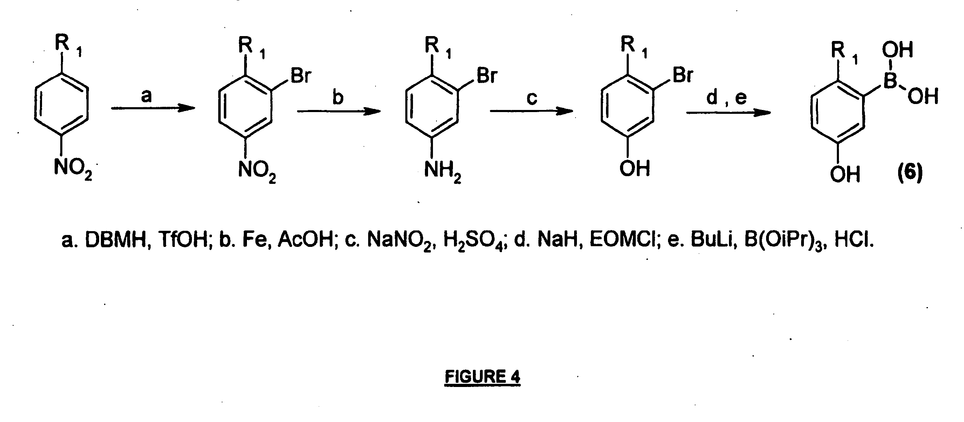

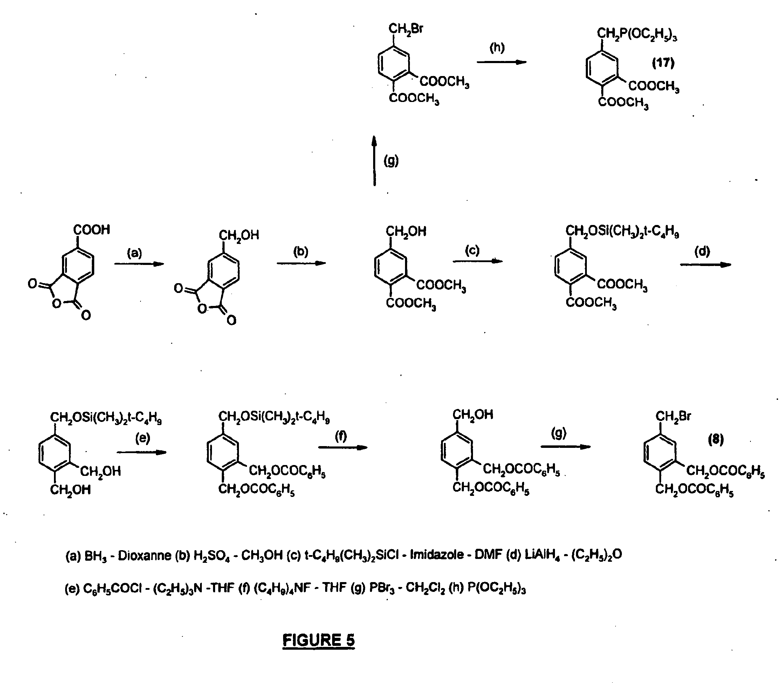

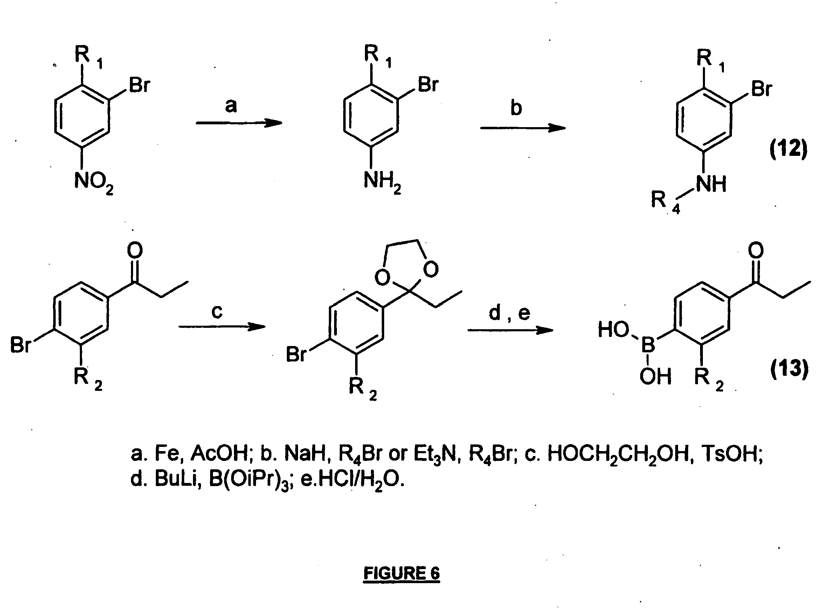

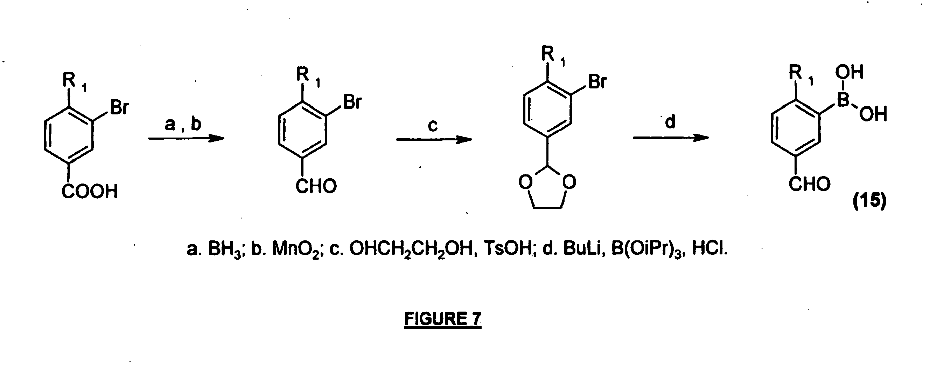

Le composé (6) peut être obtenu suivant le schéma réactionnel de la figure 4. Les composés (8) et (17) peuvent être obtenus selon le schéma réactionnel de la figure 5. Les composés (12) et (13) peuvent être obtenus suivant le schéma réactionnel de la figure 6.
Le composé (15) peut être obtenu suivant le schéma réactionnel de la figure 7.

Lorsque R₃ représente le radical trifluorométhyle, les composé (I) peuvent être obtenus à partir du dérivé bromé correspondant par formation du dérivé magnésien ou lithien puis réaction avec l'hexafluoroacétone.

Les composés selon l'invention présentent des propriétés biologiques analogues à celles de la vitamine D, notamment les propriétés de transactivation des éléments de réponse à la vitamine D (VDRE), telles qu'une activité agoniste ou antagoniste vis-à-vis de récepteurs de la vitamine D ou de ses dérivés. Par vitamines D ou leurs dérivés on entend, par exemple, les dérivés de la vitamine D₂ ou D₃ et en particulier la 1,25-dihydroxyvitamine D₃ (calcitriol).

Cette activité agoniste vis-à-vis de récepteurs de la vitamine D ou de ses dérivés peut être mise en évidence "in vitro" par des méthodes reconnues dans le domaine de l'étude de la transcription génique (Hansen et al., The Society For Investigative Dermatologie, vol.1, N°1, April 1996).

Les propriétés biologiques analogues à la vitamine D peuvent être également mesurées par la capacité du produit à induire la différenciation des cellules de leucémie promyélocytaires HL60. Le protocole ainsi que les résultats obtenus avec les composés selon l'invention sont décrits dans l'exemple 8 de la présente demande.

A titre d'exemple, l'activité agoniste VDR peut être testée sur la lignée cellulaire HeLa, par cotransfection d'un vecteur d'expression du récepteur VDR humain et du plasmide rapporteur p240Hase-CAT. L'activité agoniste peut aussi être caractérisée dans ce système de cotransfection, par la détermination de la dose nécessaire pour atteindre 50 % de l'activité maximale du produit (AC50). Le détail du protocole de ce test ainsi que les résultats obtenus avec les composés selon l'invention sont décrits dans l'exemple 9 de le présente demande.

Les propriétés biologiques analogues à la vitamine D peuvent être également mesurées par la capacité du produit à inhiber la prolifération des kératinocytes humaines normaux (KHN en culture). Le produit est ajouté à des KHN cultivés dans des conditions favorisant l'état prolifératif. Le produit est laissé au contact des cellules pendant cinq jours. Le nombre de cellules prolifératives est mesuré par incorporation de Bromodeoxyuridine (BRdU) dans l'ADN. Le protocole de ce test ainsi que les résultats obtenus avec les composés selon l'invention sont décrits dans l'exemple 10 de la présente demande.

La présente invention a également pour objet à titre de médicament les composés décrits ci-dessus.

Les composés selon l'invention conviennent particulièrement bien dans les domaines de traitement suivants :
1) Pour traiter les affections dermatologiques liées à un désordre de la différenciation ou de la prolifération des kératinocytes ou des sébocytes notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) Pour traiter des troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal) ;
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ;
4) pour traiter certaines affections inflammatoires ne présentant pas de trouble de la kératinisation, telles que l'eczéma atopique et les allergies de contact ;
5) Pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires ;
6) Pour traiter d'autres désordres dermatologiques tels que les dermatoses bulleuses et les maladies du collagène ;
7) Pour prévenir ou traiter les signes du vieillissement de la peau, qu'il soit photo-induit ou chronologique ou pour réduire les pigmentations et les kératoses actiniques, ou toutes pathologies cutanées associées au vieillissement chronologique ou actinique ;
8) Pour prévenir ou traiter les troubles de la cicatrisation ou pour prévenir ou réparer les vergetures ;
9) Pour lutter contre les troubles de la fonction sébacée tels que l'hyperséborrhée de l'acné ou la séborrhée simple ou encore l'eczéma séborrhéique ;
10) Pour traiter certains troubles ophtalmologiques, notamment les cornéopathies ;
11) Dans le traitement ou la prévention des états cancéreux ou précancéreux de cancers présentant ou pouvant être induits pour présenter des récepteurs de vitamine D, tels que, mais sans limitation, le cancer du sein, la leucémie, les syndromes myélodysplasiques et les lymphomes, les carcinomes des cellules de l'épithélium malpighien et les cancers gastro-intestinaux, les mélanomes, et l'ostéosarcome ;
12) Dans le traitement d'affections inflammatoires telles que l'arthrite ou la polyarthrite rhumatoïde ;
13) Dans le traitement de toute affection d'origine virale au niveau cutané ou général ;
14) Dans la prévention ou le traitement de l'alopécie de différentes origines, notamment l'alopécie due à la chimiothérapie ou aux rayonnements ;
15) Dans le traitement d'affections dermatologiques ou générales à composante immunologique ;
16) Dans le traitement d'affections immunitaires, telles que les maladies auto-immunes (comme, mais sans limitation, le diabète sucré de type 1, la sclérose en plaques, le lupus et les affections de type lupus, l'asthme, la glomérulonéphrite, etc.), des dysfonctionnements sélectifs du système immunitaire (par exemple le SIDA) et la prévention du rejet immun [comme les rejets de greffons (par exemple le rein, le coeur, la moelle osseuse, le foie, des îlots pancréatiques ou tout le pancréas, la peau, etc.) ou la prévention de la maladie du greffon contre l'hôte] ;
17) Dans le traitement d'affections endocriniennes étant donné que les analogues de la vitamine D peuvent moduler la sécrétion hormonale telle que l'augmentation de la sécrétion d'insuline ou la suppression sélective de la sécrétion de l'hormone parathyroïdienne (par exemple dans l'insuffisance rénale chronique et l'hyperparathyroïdie secondaire) ;
18) Dans le traitement d'affections caractérisées par une gestion anormale du calcium intracellulaire ; et
19) Dans le traitement et ou la prévention des carences en vitamine D et d'autres affections de l'homéostasie des minéraux dans le plasma et les os, tel que le rachitisme, l'ostéomalacie, l'ostéoporose, notamment dans le cas des femmes monopausées, l'ostéodystrophie rénale, les troubles de la fonction parathyroïdienne.

La présente invention a également pour objet une composition pharmaceutique comprenant au moins un composé tel que défini ci-dessus dans un support pharmaceutiquement acceptable.

L'administration des composés selon l'invention peut être effectuée par voie entérale, parentérale, topique ou oculaire.

Par voie entérale, les compositions pharmaceutiques peuvent se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée.
Par voie parentérale, les compositions peuvent se présenter sous forme de solutions ou suspensions pour perfusion ou pour injection.

Les composés selon l'invention sont généralement administrés à une dose journalière d'environ 0,001 µg/kg à 1000 µg/kg et de préférence d'environ 0,01 µg/kg à 100 µg/kg en poids corporel en 1 à 3 prises.

Par voie topique, les compositions pharmaceutiques à base de composés selon l'invention sont destinées au traitement de la peau, du cuir chevelu et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de suspensions de microsphères ou nanosphères ou de vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pour la voie topique ou oculaire contiennent au moins un composé selon l'invention à une concentration de préférence comprise entre 0,0001 % et 5 % et de préférence entre 0,001 % à 1 % par rapport au poids total de la composition.

Les composés selon l'invention trouvent également une application dans le domaine cosmétique, en particulier dans l'hygiène corporelle et capillaire et notamment pour le traitement des peaux à tendance acnéique, pour la repousse des cheveux, ou pour freiner leur chute, pour lutter contre l'aspect gras de la peau ou des cheveux, dans la protection contre les effets néfastes du soleil ou dans le traitement des peaux sèches, pour prévenir et/ou traiter le vieillissement cutané photo-induit ou chronologique.

La présente invention vise donc également une composition cosmétique contenant, dans un support cosmétiquement acceptable, au moins un composé tel que défini ci-dessus.

Cette composition cosmétique peut se présenter notamment sous forme d'une crème, d'un lait, d'une lotion, d'un gel, d'une suspension de microsphères ou nanosphères ou de vésicules lipidiques ou polymériques, d'un savon ou d'un shampooing.

La concentration en composé de formule générale (1) dans la composition cosmétique selon l'invention peut être comprise entre 0,001 et 3 % en poids par rapport au poids total de la composition.

Dans les domaines pharmaceutique et cosmétique, les composés selon l'invention peuvent être avantageusement employés en combinaison avec des additifs inertes ou même pharmacodynamiquement ou cosmétiquement actifs ou des combinaisons de ces additifs et notamment :
- des agents mouillants ;
- des agents d'amélioration de la saveur ;
- des agents conservateurs tels que les esters de l'acide parahydroxybenzoïque ;
- des agents stabilisants ;
- des agents régulateurs d'humidité ;
- des agents régulateurs de pH ;
- des agents modificateurs de pression osmotique ;
- des agents émulsionnants ;
- des filtres UV-A et UV-B ;
- des anti-oxydants tels que l'α-tocophérol, le butylhydroxyanisole, le butylhydroxytoluène, la Super Oxyde Dismutase, l'Ubiquinol ou certains chélatants de métaux ;
- des agents dépigmentants tels que l'hydroquinone, l'acide azéaïque, l'acide caféique ou l'acide kojique ;
- des émollients ;
- des agents hydratants comme le glycérol, le PEG 400, la thiamorpholinone et ses dérivés ou l'urée;
- des agents antiséborrhéiques ou antiacnéiques, tels que la S-carboxyméthylcystéine, la S-benzyl-cystéamine, leurs sels et leurs dérivés, ou le péroxyde de benzoyle ;
- des antibiotiques comme l'érythromycine et ses esters, la néomycine, la clindamycine et ses esters, les tétracyclines ;
- des agents antifongiques tels que le kétoconazole ou les polyméthylène-4,5 isothiazolinones-3 ;
- des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphényl-imidazolidine 2,4-dione) ;
- des agents anti-inflammatoires non stéroïdiens ;
- des caroténoïdes et, notamment le β-caroténe ;
- des agents anti-psoriatiques tels que l'anthraline et ses dérivés ;
- les acides eicosa-5,8,11,14- tétraynoïque et eicosa-5,8,11-trynoïque, leurs esters et les amides ;
- des rétinoides, c'est à dire des ligands des récepteurs RAR ou RXR, naturels ou synthétiques ;
- des corticostéroïdes ou des oestrogènes;
- des α-hydroxy acides et des α-céto acides ou leurs dérivés, tels que les acides lactique, malique, citrique, glycolique, mandélique, tartrique, glycérique, ascorbique, ainsi que leurs sels, amides ou esters, ou des β-hydroxy acides ou leurs dérivés, tels que l'acide salicylique ainsi que ses sels, amides ou esters ;
- des bloqueurs de canaux ioniques tels que les canaux potassiques ;
- ou encore, plus particulièrement pour les compositions pharmaceutiques, en association avec des médicaments connus pour interférer avec le système immunitaire (par exemple, la cyclosporine, le FK 506, les glucocorticoïdes, les anticorps monoclonaux, les cytokines ou les facteurs de croissance...).

Bien entendu, l'homme du métier veillera à choisir le ou les éventuels composés à ajouter à ces compositions de telle manière que les propriétés avantageuses des composés de la présente invention ne soient pas ou substantiellement pas altérées par l'addition envisagée.

On va maintenant donner, à titre d'illustration et sans aucun caractère limitatif, des exemples d'obtention des composés actifs selon l'invention tels que cités plus haut, ainsi que diverses formulations concrètes à base de tels composés et des tests d'évaluation de l'activité biologique des composés selon l'invention.

### EXEMPLE 1: {5-[4'-(1-Ethyl-1-hydroxy-propyl)-6-methyl-2'-propyl-biphenyl-3-yloxymethyl]-2-hydroxymethyl-phenyl}-methanol.

### (a) 1-(4-Hydroxy-3-propyl-phenyl)-propan-1-one.

Dans un ballon et sous courant d'azote, on introduit 7,1 g (53 mmoles) de chlorure d'aluminium dans 70 ml de nitrobenzène. Le mélange est chauffé à 70°C jusqu'à complète dissolution. Il est ensuite refroidi à 0°C et 7,2 ml (52 mmoles) de 2-propylphénol sont rajoutés. On laisse remonter à température ambiante puis on chauffe à 40°C. 4,6 ml (52 mmoles) de chlorure de propionyle sont alors additionnés, goutte à goutte. Le milieu réactionnel est chauffé à 40°C pendant 2 heures puis est agité 48 heures à température ambiante. Il est alors versé dans de la glace avec 20 ml d'acide chlorhydrique concentré. Après extraction avec de l'éther éthylique, la phase organique est lavée avec de la soude 2N. Les phases aqueuses sont acidifiées avec de l'acide chlorhydrique et extraites à l'éther. La phase organique est séchée sur sulfate de magnésium, filtrée puis évaporée. Le solide noir obtenu est trituré dans de l'heptane, filtré et séché. On obtient 3,5 g (35%) du produit attendu sous la forme d'une poudre noire.

### (b) 1,1,1-Trifluoro-methanesulfonafe de 4-propionyl-2-propyl-phenyle.

Dans un ballon et sous courant d'azote, on introduit 3,4 g (18 mmoles) de 1-(4-Hydroxy-3-propyl-phenyl)-propan-1-one dans 100 ml de dichlorométhane. Le milieu réactionnel est refroidi à 0°C puis 3,3 ml (23 mmoles) de triéthylamine sont ajoutés. 15 minutes après, on rajoute 4,3 ml (26 mmoles) d'anhydride trifluorométhanesulfonique. 1 heure après, le milieu est versé dans une solution aqueuse saturée en chlorure d'ammonium et extrait avec du dichlorométhane. La phase organique est séchée sur sulfate de magnésium, filtrée puis évaporée. Le résidu obtenu est purifié par chromatographie sur colonne de silice élué avec un mélange d'heptane et d'acétate d'éthyle (70/30). Après évaporation des solvants, on obtient 5,4 g (93%) du produit attendu sous la forme d'une huile marron.

### (c) 3-Bromo-4-methyl-phenylamine.

Dans un ballon et sous courant d'azote, on introduit 30 g (139 mmoles) de 2-bromo-4-nitrotoluène dans 180 ml d'eau, 400 ml d'éthanol et 110 ml d'acide acétique. Le milieu est chauffé à 70°C et on ajoute, par petites fractions, 31 g (556 mmoles) de fer. On chauffe au reflux pendant 2 heures, puis, après refroidissement, on rajoute, lentement, 180 ml d'ammoniaque à 34%. Le mélange est filtré sur célite et la phase organique est extrait avec de l'eau et de l'acétate d'éthyle. Elle est ensuite séchée sur sulfate de magnésium, filtrée puis évaporée. On obtient 25,5 g (100%) du produit attendu sous la forme d'une huile marron.

### (d) 3-Bromo-4-methyl-phenol.

Dans un ballon, on introduit 25 g (134 mmoles) de 2-bromo-4-aminotoluène dans 400 ml d'acide sulfurique 1 M puis le mélange est refroidi à 0°C. 13 g (190 mmoles) de nitrite de sodium en solution dans 30 ml d'eau sont rajoutés puis 21 ml d'acide sulfurique concentré. Le mélange est chauffé à reflux pendant 4 heures. Il est, ensuite, extrait à l'éther éthylique. La phase organique est séchée sur sulfate de magnésium, filtrée puis évaporée. Le résidu obtenu est purifié par chromatographie sur colonne de silice élué avec un mélange d'heptane et d'acétate d'éthyle (90/10) et on obtient 12,1 g (48%) du produit attendu sous la forme d'une huile marron.

### (e) 2-Bromo-4-ethoxymethoxy-1-methyl-benzene.

Dans un ballon et sous courant d'azote, on introduit 12,1 g (65 mmoles) de 3-bromo-4-methyl-phenol dans 100 ml de diméthylformamide sec. A 0°C. 3,1 g (78 mmoles) d'hydrure de sodium à 60% sont additionnés lentement. 1 heure après, 7,3 ml (78 mmoles) de chlorure de méthoxyéthyle sont ajoutés, goutte à goutte. Le mélange est agité à température ambiante une nuit. Il est, alors, versé dans de l'eau et extrait à l'acétate d'éthyle. La phase organique est séchée sur sulfate de magnésium, filtrée puis évaporée. Le résidu obtenu est purifié par chromatographie sur colonne de silice élué avec un mélange d'heptane et d'acétate d'éthyle (85/15) et on obtient 13,2 g (83%) du produit attendu sous la forme d'une huile.

### (f) Acide 5-ethoxymethoxy-2-methyl-phénylboronique.

Dans un ballon et sous courant d'azote, on introduit 1,4 g (59 mmoles) de tournures de magnésium dans du tétrahydrofurane. On additionne lentement 13,2 g (54 mmoles) de 2-Bromo-4-ethoxymethoxy-1-methyl-benzene dilués dans un peu de THF. Le milieu est porté au reflux pendant 20 minutes. Il est ensuite rajouté au moyen d'une canule à 15 ml (65 mmoles) de borate de triisopropyle. Le mélange prend en masse et est alors versé dans une solution d'acide chlorhydrique 1 N et extrait à l'éther. La phase organique est séchée sur sulfate de magnésium, filtrée puis évaporée. On obtient 9,8 g (87%) du produit attendu sous la forme d'une huile marron.

### (g) 1-(5'-Ethoxymethoxy-2'-methyl-2-propyl-biphenyl-4-yl)-propan-1-one.

Dans un ballon et sous courant d'azote, 1,56 g (7,4 mmoles) d'acide 5-ethoxymethoxy-2-methyl-phénylboronique, 2 g (6,2 mmoles) de 1,1,1-Trifluoro-methanesulfonate de 4-propionyl-2-propyl-phenyle, 517 mg (12 mmoles) de chlorure de lithium et 7,4 ml d'une solution de carbonate de potassium 2M sont dissous dans 50 ml de 1,2-diméthoxyéthane. Le mélange est porté au reflux pendant 10 heures. Il est ensuite versé dans de l'eau et extrait à l'acétate d'éthyle. La phase organique est séchée sur sulfate de magnésium, filtrée puis évaporée. On obtient une huile marron qui est utilisée telle quelle pour la suite de la synthèse.

### (h) 1-(5'Hydroxy-2'methyl-2-propyl-biphenyl-4-yl)-propan-1-one. CS 755.064

Le 1-(5'-Ethoxymethoxy-2'-methyl-2-propyl-biphenyl-4-yl)-propan-1-one, obtenu à l'étape (g), est dissous dans du méthanol et on ajoute quelques gouttes d'acide sulfurique. L'agitation est maintenue toute la nuit puis le milieu est extrait avec de l'acétate d'éthyle et de l'eau. La phase organique est séchée sur sulfate de magnésium, filtrée puis évaporée. Le résidu obtenu est purifié par chromatographie sur colonne de silice élué avec un mélange d'heptane et d'acétate d'éthyle (75/25) et on obtient 1,53 g (88%) du produit attendu sous la forme d'une huile marron.

### (i) 4-Hydroxymethyl-phthalate de diméthyle.

Dans un ballon et sous courant d'azote, on introduit 50 g (260 mmoles) d'anhydride triméllitique dans 500 ml de dioxane. On ajoute, goutte à goutte, 520 ml (520 mmoles) de borane (1M/THF) et agite 48 heures à température ambiante. Le milieu réactionnel est, alors, versé lentement dans une solution aqueuse saturée en chlorure d'ammonium et extrait au dichlorométhane. La phase organique est séchée sur sulfate de magnésium, filtrée puis évaporée. Le résidu est dissous dans 400 ml de méthanol et 5 ml d'acide sulfurique sont rajoutés. On chauffe à 80°C pendant une nuit. Le méthanol est évaporé et le résidu est repris par de l'acétate d'éthyle et lavé à l'eau. La phase organique est séchée sur sulfate de magnésium, filtrée puis évaporée. On obtient 52,7 g (90%) du produit attendu sous la forme d'une huile jaune.

### (j) 4-(tert-Butyl-dimethyl-silanyloxymethyl)-phthalate de diméthyle.

Dans un ballon et sous courant d'azote, on introduit 19 g (84,8 mmoles) de 4-hydroxymethyl-phthalate de diméthyle dans 250 ml de diméthylformamide. On ajoute 14 g (93 mmoles) de chlorure de *tert*-butyldiméthylsilane et 8 g (118 mmoles) d'imidazole. Le milieu réactionnel est agité 3 heures à température ambiante, il est, ensuite concentré et le résidu est dissous dans de l'éther puis filtré. Le filtrat est évaporé et le produit est purifié par chromatographie sur colonne de silice élué avec un mélange d'heptane et d'acétate d'éthyle (50/50) et on obtient 23,7 g (83%) du produit attendu sous la forme d'une huile jaune.

### (k) [4-(tert-Butyl-dimethyl-silanyloxymethyl)-2-hydroxymethyl-phenyl]-mothanol.

Dans un ballon et sous courant d'azote, on introduit 17,8 g (469 mmoles) d'hydrure de lithiumaluminium dans 800 ml d'éther éthylique. A 0°C, on ajoute, goutte à goutte, une solution de 66,4 g (196 mmoles) de 4-(*tert*-Butyl-dimethyl-silanyloxymethyl)-phthalate de diméthyle dans 200 ml d'éther et 100 ml de THF. Le milieu réactionnel est agité à 0°C pendant 2 heures. Très lentement, on ajoute 18 ml d'eau, 18 ml d'une solution de soude à 15%, puis 54 ml d'eau. Le précipité formé est filtré, rincé à l'éther et le filtrat est évaporé. On obtient 52 g (94%) du produit attendu sous la forme d'une huile incolore.

### (I) Benzoate de 2-benzoyloxyméthyl-4-(tert-butyl-diméthyl-silanyloxyméthyl) benzyle.

Dans un ballon et sous courant d'azote, on introduit 40 g (141 mmoles) de [4-(*tert*-Butyldimethyl-silanyloxymethyl)-2-hydroxymethyl-phenyl]-methanol dans 400 ml de THF. A 0°C, on ajoute 49 ml (352 mmoles) de triéthylamine puis, goutte à goutte, 34,5 ml (297 mmoles) de chlorure de benzoyle. Le milieu réactionnel précipite et on ajoute alors 350 mg (2,8 mmoles) de diméthylaminopyridine. On laisse remonter à température ambiante la nuit. Le milieu est filtré et le solide lavé avec de l'acétate d'éthyle. Le filtrat est évaporé et le résidu est repris par du dichlorométhane. La phase organique est lavée avec une solution aqueuse saturée en chlorure d'ammonium puis avec de l'eau. Elle est ensuite séchée sur sulfate de magnésium, filtrée et évaporée. On obtient 69,5 g (100%) du produit attendu sous la forme d'une huile orangée.

### (m) Benzoate de 2-benzoyloxyméthyl-4-hydroxyméthyl)benzyle.

Dans un ballon et sous courant d'azote, on introduit 69 g (140 mmoles) de benzoate de 2-benzoyloxyméthyl-4-(*tert*-butyl-diméthyl-silanyloxyméthyl)benzyle dans 450 ml d'acétate d'éthyle. On ajoute 178 ml de fluorure de tétrabutylammonium (1M/THF). Le milieu est agité 30 minutes à température ambiante. Il est ensuite versé dans une solution aqueuse saturée en chlorure d'ammonium et extrait à l'acétate d'éthyle. La phase organique est séchée sur sulfate de magnésium, filtrée puis évaporée. Le résidu est purifié par chromatographie sur colonne de silice élué avec un mélange heptane et acétate d'éthyle (40/60) et on obtient 46,6 g (88%) du produit attendu sous la forme d'une poudre blanche de point de fusion 92°C.

### (n) Bromure de (3,4-bis-benzoyloxyméthyl)benzyle.

Dans un ballon et sous courant d'azote, on introduit 40 g (106 mmoles) de benzoate de 2-benzoyloxyméthyl-4-hydroxyméthyl)benzyle dans 500 ml de dichlorométhane et 77,6 g (234 mmoles) de tétrabromure de carbone. A 0°C, on ajoute, goutte à goutte, une solution de 61,3 g (233 mmoles) de triphénylphosphine dans 200 ml de dichlorométhane. On laisse remonter à température ambiante pendant 2 heures. Le milieu réactionnel est versé dans de l'eau et extrait au dichlorométhane. La phase organique est séchée sur sulfate de magnésium, filtrée puis évaporée. Le résidu est purifié par chromatographie sur colonne de silice élué avec un mélange heptane et acétate d'éthyle (70/30) et on obtient 32,6 g (70%) du produit attendu sous la forme d'une poudre blanche.

### (o) 1-[5'=(3,4-Bis-benzoyloxymethyl-benzyloxy)-2'-methyl-2-propyl-biphenyl-4-yl]-propan-1-one.

Dans un ballon et sous courant d'azote, on introduit 1,5 g (5,4 mmoles) de 1-(5'-Hydroxy-2'-methyl-2-propyl-biphenyl-4-yl)-propan-1-one, 2,5 g (5,7 mmoles) de bromure de (3,4-bis-benzoyloxyméthyl)benzyle et 750 mg (5,4 mmoles) de carbonate de potassium dans 50 ml de méthyléthylcétone. Le mélange est porté au reflux toute une nuit, puis, après refroidissement il est filtré sur célite. Le filtrat est extrait avec de l'eau et de l'acétate d'éthyle. La phase organique est séchée sur sulfate de magnésium, filtrée puis évaporée. Le résidu obtenu est purifié par chromatographie sur colonne de silice élué avec un mélange d'heptane et d'acétate d'éthyle (85/15) et on obtient 1,93 g (56%) du produit attendu sous la forme d'une huile jaune foncé.

### (p) 1-[5'-(3,4-Bis-hydroxymethyl-benzyloxy)-2'-methyl-2-propyl-biphenyl-4-yl]-propan-1-one.

Dans un ballon, 1,9 g (3 mmoles) de 1-[5'-(3,4-Bis-benzoyloxymethyl-benzyloxy)-2'-methyl-2-propyl-biphenyl-4-yl]-propan-1-one sont dissous dans 40 ml d'une solution méthanolique à 2% de carbonate de potassium. Le mélange est agité à température ambiante toute une nuit. Il est, ensuite, versé dans de l'eau et extrait à l'acétate d'éthyle. La phase organique est séchée sur sulfate de magnésium, filtrée puis évaporée. Le résidu obtenu est purifié par chromatographie sur colonne de silice élué avec un mélange d'heptane et d'acétate d'éthyle (70/30) et on obtient 890 mg (69%) du produit attendu.

### (q) {5-[4'(1-Ethyl-1-hydroxy-propyl)-6-methyl-2'-propyl-biphenyl-3-yloxymethyl]-2-hydroxymethyl-phenyl}-methanol.

Dans un ballon et sous courant d'azote, on introduit 890 mg (2 mmoles) de 1-[5'-(3,4-Bis-hydroxymethyl-benzyloxy)-2'-methyl-2-propyl-biphenyl-4-yl]-propan-1-one dans 40 ml de THF sec. A 0°C, on rajoute 4,1 ml (12 mmoles) de bromure d'éthylmagnésium. A température ambiante, le milieu réactionnel est agité 1 heure 30 puis est versé dans une solution aqueuse saturée en chlorure d'ammonium et extrait à l'acétate d'éthyle. La phase organique est séchée sur sulfate de magnésium, filtrée puis évaporée. Le résidu obtenu est purifié par chromatographie sur colonne de silice élué avec un mélange d'heptane et d'acétate d'éthyle (50/50) et on obtient 550 mg (64%) de {5-[4'-(1-Ethyl-1-hydroxy-propyl)-6-methyl-2'-propyl-biphenyl-3-yloxymethyl]-2-hydroxymethyl-phenyl}-methanol sous la forme de cristaux blancs de point de fusion 97°C.

### EXEMPLE 2: {5-[6,2'-Diethyl-4'-(1-ethyl-1-hydroxy-propyl)-biphenyl-3-yloxymethyl]-2-hydroxymethyl-phenyl}-methanol.

### (a) 4-Bromo-2-ethy/-pheno/.

Dans un ballon et sous courant d'azote, on introduit 5,57 g (45 mmoles) de 2-éthylphénol dans 250 ml de chloroforme. On ajoute, en petites fractions, 43,4 g (90 mmoles) de tetrabutylammonium tribromide. Le mileu est agité 1 heure à température ambiante, puis, il est hydrolysé avec une solution aqueuse saturée en thiosulfate de sodium. La phase organique est lavée à l'eau, séchée sur sulfate de magnésium, filtrée puis évaporée. On obtient 9,1 g (100%) du produit attendu sous la forme d'une huile jaune.

### (b) (4-Bromo-2-ethyl-phenoxy)-tert-butyl-dimethyl-silane.

De manière analogue à l'exemple 1(j), à partir de 9,1 g (45 mmoles) de 4-Bromo-2-ethylphenol, on obtient, après purification par chromatographie sur colonne de silice élué avec de l'heptane 11,9 g (83%) du produit attendu sous la forme d'une huile incolore.

### (c) 1-[4-(tert-Butyl-dimethyl-silanyloxy)-3-ethyl-phenyl]-propan-1-ol.

Dans un ballon et sous courant d'azote, on introduit 11,9 g (38 mmoles) de (4-Bromo-2-ethyl-phenoxy)-tert-butyl-dimethyl-silane dans 200 ml de THF. Le milieu réactionnel est refroidi à -78°C et on ajoute, goutte à goutte, 16,6 ml (41 mmoles) de n-Butyllithium (2,5 M/THF). 30 minutes après, on ajoute, goutte à goutte, 3,2 ml (45 mmoles) d'aldéhyde propionique. Le milieu est agité pendant 3 heures à -78°C. Il est, ensuite, versé dans une solution aqueuse saturée en chlorure d'ammonium et extrait à l'acétate d'éthyle. La phase organique est séchée sur sulfate de magnésium, filtrée puis évaporée. On obtient 11,1 g (100%) du produit attendu sous la forme d'une huile incolore.

### (d) 1-[4-(tert-Butyl-dimethyl-silanyloxy)-3-ethyl-phenyl]-propan-1-one.

Dans un ballon, on introduit 11,1 g (37 mmoles) de 1-[4-(tert-Butyl-dimethyl-silanyloxy)-3-ethyl-phenyl]-propan-1-ol dans 100 ml de dichlorométhane et on ajoute 32,8 g (377 mmoles) de dioxyde de manganèse. Le milieu est agité une nuit à température ambiante. Il est alors filtré sur célite et rincé avec du dichlorométhane. Le filtrat est évaporé et le résidu est purifié par chromatographie sur colonne de silice élué avec un mélange d'heptane et acétate d'éthyle (90/10) et on obtient 6,8 g (62%) du produit attendu sous la forme d'une huile orangée.

### (e) 1-(3-Ethyl-4-hydroxy-phenyl)-propan-1-one.

De manière analogue à l'exemple 1(m), à partir de 6,8 g (23 mmoles) de 1-[4-(tert-Butyldimethyl-silanyloxy)-3-ethyl-phenyl]-propan-1-one dans 50 ml de THF, on obtient 4,1 g (100%) du produit attendu sous la forme d'une huile beige:

### (f) 1,1,1-Tritluoro-methanesulfonate de 2-ethyl-4-propiony/-pheny/e.

De manière analogue à l'exemple 1(b), par réaction de 4,6 g (26,3 mmoles) de 1-(3-Ethyl-4-hydroxy-phenyl)-propan-1-one avec 4,8 ml (28,9 mmoles) d'anhydride triftuorométhanesulfonique, on obtient 8,1 g (99%) du produit attendu sous la forme d'une huile marron.

### (g) 1-bromo-2-éthyl-5-nitro-benzene.

Dans un ballon et sous courant d'azote, on introduit 25 g (165 mmoles) de 4-ethylnitrobenzene dans 200 ml de dichlorométhane. On ajoute 20 ml (230 mmoles) d'acide trifluorométhanesulfonique et 33 g (115 mmoles) de 1,3-dibromo-5,5-dimethyl hydantoin et le milieu réactionnel est agité 2 heures à température ambiante. On ajoute alors une solution aqueuse saturée en sodium hydrosulfite. Les phases sont séparées et la phase organique est neutralisée avec une solution aqueuse 2M de carbonate de sodium, puis est lavée à l'eau. Elle est, ensuite, séchée sur sulfate de magnésium, filtrée et évaporée. On obtient 35 g (92%) du produit attendu sous la forme d'une huile jaune.

### (h) 3-bromo-4-ethyl-phenylamine.

De manière analogue à l'exemple 1(c), par réaction de 34 g (148 mmoles) de 1-bromo-2-éthyl-5-nitro-benzene avec 33 g (591 mmoles) de fer, on obtient, après purification par chromatographie sur colonne de silice élué avec un mélange d'heptane et d'acétate d'éthyle (90/10), 27 g (89%) du produit attendu sous la forme d'une huile jaune.

### (i) 3-bromo-4-ethyl-phenol.

De manière analogue à l'exemple 1(d), par réaction de 27 g (135 mmoles) 3-bromo-4-ethyl-phenylamine avec 11 g (162 mmoles) de nitrite de sodium, on obtient, après purification par chromatographie sur colonne de silice élué avec un mélange d'heptane et d'acétate d'éthyle (90/10), 13 g (49%) du produit attendu sous la forme d'une huile marron.

### (j) 1-bromo-2-éthyl-5-ethoxymethoxy-benzene.

De manière analogue à l'exemple 1(e), par réaction de 13 g (65 mmoles) 3-bromo-4-ethylphenol avec 6,7 ml (72 mmoles) de chlorure de methoxyethyle, on obtient, après purification par chromatographie sur colonne de silice élué avec un mélange d'heptane et d'acétate d'éthyle (90/10), 12g (76%) du produit attendu sous la forme d'une huile jaune.

### (k) Acide 2-éthyl-5-ethoxymethoxy-phénylboronique.

Dans un ballon et sous courant d'azote, on introduit 12 g (49 mmoles) de 1-bromo-2-éthyl-5-ethoxymethoxy-benzene dans 200 ml de THF sec. Le milieu réactionnel est refroidi à - 78°C et on ajoute, goutte à goutte, 23 ml (58 mmoles) de n-Butyllithium 2,5 M/hexane. 20 minutes après, 13,6 ml (59 mmoles) de borate de triisopropyle sont additionnés lentement. L'agitation est maintenue 1 heure à -78°C. Le mélange est alors versé dans une solution d'eau et d'acide chlorhydrique, puis, est extrait à l'acétate d'éthyle. La phase organique est séchée sur sulfate de magnésium, filtrée et évaporée. Le résidu obtenu est purifié par chromatographie sur colonne de silice élué avec un mélange d'heptane et d'acétate d'éthyle (50/50). Après évaporation des solvants, on obtient 6,7 g (65%) du produit attendu sous la forme de cristaux blancs.

### (I) 1-(5'-Ethoxymethoxy-2,2'-diethyl-biphenyl-4-yl)-propan-1-one.

De manière analogue à l'exemple 1(g), par réaction de 2 g (8,9 mmoles) d' acide 2-éthyl-5-ethoxymethoxy-phénylboronique avec 2,1 g (6,9 mmoles) de 1,1,1-Trifluoro-methanesulfonate de 2-ethyl-4-propionyl-phenyle, on obtient une huile noire qui est utilisée telle quelle pour la suite de la synthèse.

### (m) 1-(2,2'-Diethyl-5-hydroxy-biphenyl-4-yl)-propan-1-one.

De manière analogue à l'exemple 1(h), à partir de 1-(5'-Ethoxymethoxy-2,2'-diethylbiphenyl-4-yl)-propan-1-one, on obtient après purification par chromatographie sur colonne de silice élué avec un mélange d'heptane et d'acétate d'éthyle (50/50), 1,6 g (86%) du produit attendu sous la forme d'une huile jaune.

### (n) 1-[5'(3,4-Bis-benzoyloxymethyl-benzyloxy)-2,2'-diethyl-biphenyl-4-yl]-propan-1-one.

De manière analogue à l'exemple 1(o), par réaction de 1,67 g (5,9 mmoles) de 1-(2,2'-Diethyl-5'-hydroxy-biphenyl-4-yl)-propan-1-one avec 2,73 g (6,2 mmoles) de bromure de (3,4-bis-benzoyloxyméthyl)benzyle, on obtient après purification par chromatographie sur colonne de silice élué avec un mélange d'heptane et d'acétate d'éthyle (95/5), 1,88 g (50%) du produit attendu sous la forme d'une huile.

### (o) 1-[5'-(3,4-Bis-hydroxymethy/-benzy/oxy)-2,2'-diethy/-biphenyl-4-yl]-propan-1-one.

De manière analogue à l'exemple 1(p), à partir de 1,8 g (2,9 mmoles) 1-[5'-(3,4-Bis-benzoyloxymethyl-benzyloxy)-2,2'-diethyl-biphenyl-4-yl]-propan-1-one, on obtient après purification par chromatographie sur colonne de silice élué avec un mélange d'heptane et d'acétate d'éthyle (40/60), 840 mg (68%) du produit attendu sous la forme d'une huile incolore.

### (p){5-[6,2'-Diethyl-4'-(1-ethyl-1-hydroxy-propyl)-biphenyl-3-yloxymethyl]-2-hydroxymethyl-phenyl}-methanol.

De manière analogue à l'exemple 1(q), par réaction de 840 mg (1,9 mmoles) de 1-[5'-(3,4-Bis-hydroxymethyl-benzyloxy)-2,2'-diethyl-biphenyl-4-yl]-propan-1-one avec 5,2 ml (16 moles) de bromure d'éthylmagnésium (3M/ether), on obtient après purification par chromatographie sur colonne de silice élué avec un mélange d'heptane et d'acétate d'éthyle (40/60), 254 mg (29%) de {5-[6,2'-Diethyl-4'-(1-ethyl-1-hydroxy-propyl)-biphenyl-3-yloxymethyl]-2-hydroxymethyl-phenyl}-methanol sous la forme de cristaux blancs de point de fusion 93°C.

### EXEMPLE 3: {4-[6-Ethyl-4'-(1-ethyl-1-hydroxy-propyl)-2'-propyl-biphenyl-3-yloxymethyl]-2-hydroxymethyl-phenyl}-methanol.

### (a) 1-(5'-Ethoxymethoxy-2'-ethyl-2-propyl-biphenyl-4-yl)-propan-1-one.

De manière analogue à l'exemple 1(g), par réaction de 1,58 g (7,1 mmoles) d'acide 2-éthyl-5-ethoxymethoxy-phénylboronique obtenu en 2(j) avec 1,76 g (5,4 mmoles) de 1,1,1-Trifluoro-methanesulfonate de 4-propionyl-2-propyl-phenyle obtenu en 1(b), on obtient une huile marron qui est utilisée sans purification.

### (b) 1-(2'Ethyl-5'-hydroxy-2-propyl-biphenyl-4-yl)-propan-1-one.

De manière analogue à l'exemple 1(h), à partir de 1-(5'-Ethoxymethoxy-2'-ethyl-2-propylbiphenyl-4-yl)-propan-1-one, on obtient après purification par chromatographie sur colonne de silice élué avec un mélange d'heptane et d'acétate d'éthyle (90/10), 1,28 g (80%) du produit attendu sous la forme d'une huile.

### (c) 1-[5'-(3,4-Bis-benzo/oxymethy/-benzy/oxy)-2'-ethyl-2-propy/-bipheny/-4-y/]-propan-1-one.

De manière analogue à l'exemple 1(o), par réaction de 1,28 g (4,3 mmoles) de 1-(2'-Ethyl-5'-hydroxy-2-propyl-biphenyl-4-yl)-propan-1-one avec 1,98 g (4,5 mmoles) de bromure de (3,4-bis-benzoyloxyméthyl)benzyle, on obtient une huile qui est utilisée sans purification.

### (d) 1-[5'-(3,4-Bis-hydroxymethyl-benzyloxy)-2'-ethyl-2-propyl-biphenyl-4-yl]-propan-1-one.

De manière analogue à l'exemple 1(p), à partir de 1-[5'-(3,4-Bis-benzoyloxymethylbenzyloxy)-2'-ethyl-2-propyl-biphenyl-4-yl]-propan-1-one, on obtient après purification par chromatographie sur colonne de silice élué avec un mélange d'heptane et d'acétate d'éthyle (30/70), 830 mg (43%) du produit attendu sous la forme d'une huile incolore.

### (e) {4-[6-Ethyl-4'-(1-ethyl-1-hydroxy-propyl)-2'-propyl-biphenyl-3-yloxymethyl]-2-hydroxymethyl-phenyl}-methanol.

De manière analogue à l'exemple 1(q), par réaction de 830 mg (1,9 mmole) de 1-[5'-(3,4-Bis-hydroxymethyl-benzyloxy)-2'-ethyl-2-propyl-biphenyl-4-yl]-propan-1-one avec 5,1 ml (15,2 mmoles) de bromure d'éthylmagnésium (3M/éther), on obtient, après purification par chromatographie sur colonne de silice élué avec un mélange d'heptane et d'acétate d'éthyle (40/60), 700 mg (77%) de {4-[6-Ethyl-4'-(1-ethyl-1-hydroxy-propyl)-2'-propylbiphenyl-3-yloxymethyl]-2-hydroxymethyl-phenyl]-methanol sous la forme de cristaux blancs de point de fusion 101°C.

### EXEMPLE 4: {4-[6-Ethyl-4'-(1-ethyl-1-hydroxy-propyl)-2'-isopropyl-biphenyl-3-yloxymethyl]-2-hydroxymethyl-phenyl}-methanol.

### (a) 1-(4-Hydroxy-3-isopropyl-phenyl)-propan-1-one.

De manière analogue à l'exemple 1(a), par réaction de 10 g (73 mmoles) de 2-isopropylphenol avec 6,3 ml (73 mmoles) de chlorure de propionyle, on obtient 4,5 g (32%) du produit attendu sous la forme de cristaux blancs de point de fusion 105°C.

### (b) 1,1,1-Trifluoro-methanesulfonate de 2-isopropyl-4-propionyl-phenyle.

De manière analogue à l'exemple 1(b), par réaction de 4,5 g (24 mmoles) de 1-(4-Hydroxy-3-isopropyl-phenyl)-propan-1-one avec 4,4 ml (26 mmoles) d'anhydride trifluorométhanesulfonique, on obtient 7,5 g (100%) du produit attendu.

### (c) 1-(5'-Ethoxymethoxy-2'-ethyl-2-isopropyl-biphenyl-4-yl)-propan-1-one.

De manière analogue à l'exemple 1(g), par réaction de 1,8 g (8 mmoles) d'acide 2-éthyl-5-ethoxymethoxy-phénylboronique obtenu en 2(j) avec 2 g (6,2 mmoles) de 1,1,1-Trifluoro-methanesulfonate de 2-isopropyl-4-propionyl-phenyle, on obtient une huile marron qui est utilisée sans purification.

### (d) 1-(2'-Ethyl-5'-hydroxy-2-isopropyl-biphenyl-4-yl)-propan-1-one.

De manière analogue à l'exemple 1(h), à partir de 1-(5'-Ethoxymethoxy-2'-ethyl-2-isopropyl-biphenyl-4-yl)-propan-1-one, on obtient, après purification par chromatographie sur colonne de silice élué avec un mélange d'heptane et d'acétate d'éthyle (80/20), 660 mg (35%) du produit attendu sous la forme d'une huile incolore.

### (e) 1-[5'-(3,4-Bis-benzoyloxymethyl-benzyloxy)-2'-ethyl-2-isopropyl-biphenyl-4-yl]-propan-1-one.

De manière analogue à l'exemple 1(o), par réaction de 660 mg (2,2 mmoles) de 1-(2'-Ethyl-5'-hydroxy-2-isopropyl-biphenyt-4-yl)-propan-1-one avec 1 g (2,3 mmoles) de bromure de (3,4-bis-benzoyloxyméthyl)benzyle, on obtient une huile qui est utilisée sans purification.

### (f) 1-[5'-(3,4-Bis-hydroxymethyl-benzyloxy)-2'-ethyl-2-isopropyl-biphenyl-4-yl]-propan-1-one.

De manière analogue à l'exemple 1(p), à partir de 1-[5'-(3,4-Bis-benzoyloxymethylbenzyloxy)-2'-ethyl-2-isopropyl-biphenyl-4-yl]-propan-1-one, on obtient, après purification par chromatographie sur colonne de silice élué avec un mélange d'heptane et d'acétate d'éthyle (50/50), 810 mg (83%) du produit attendu sous la forme d'une huile incolore.

### (g) {4-[6-Ethyl-4'-(1-ethyl-1-hydroxy-propyl)-2'-isopropyl-biphenyl-3-yloxymethyl]-2-hydroxymethyl-phenyl-methanol.

De manière analogue à l'exemple 1(q), par réaction de 810 mg (1,8 mmole) de 1-[5'-(3,4-Bis-hydroxymethyl-benzyloxy)-2'-ethyl-2-isopropyl-biphenyl-4-yl]propan-1-one avec 4,8 ml (15 mmoles) de bromure d'éthylmagnésium (3M/éther), on obtient, après purification par chromatographie sur colonne de silice élué avec un mélange d'heptane et d'acétate d'éthyle (35/65), 600 mg (70%) de {4-[6-Ethyl-4'-(1-ethyl-1-hydroxy-propyl)-2'-isopropylbiphenyl-3-yloxymethyl]-2-hydroxymethyl-phenyl}-methanol sous la forme de cristaux blancs de point de fusion 109°C.

### EXEMPLE 5: {2-[4'-(1-Ethyl-1-hydroxy-propyl)-6-methyl-2'-propyl-biphenyl-3-yl]-ethyl}-2-hydroxymethyl-phenyl)-methanol.

### (a) (3-Bromo-4-methyl-phenyl)-methanol.

Dans un ballon et sous courant d'azote, on introduit 15 g (70 mmoles) de l'acide 3-bromo-4-methylbenzoique dans 150 ml de THF anhydre. On ajoute, goutte à goutte, 84 ml de borane 1M/THF et le milieu réactionnel est agité une nuit à température ambiante. Une solution THF/eau (50/50) est rajoutée lentement à 0°C puis le mélange est versé dans une solution aqueuse saturée en chlorure d'ammonium et extrait à l'acétate d'éthyle. La phase organique est séchée sur sulfate de magnésium, filtrée puis évaporée. On obtient 14,4 g (100%) du produit attendu sous la forme d'une huile jaune.

### (b) 3-Bromo-4-methyl-benzaldehyde.

Dans un ballon, on introduit 14,4 g (72 mmoles) de (3-Bromo-4-methyl-phenyl)-methanol dans 200 ml de dichlorométhane. On rajoute 62,6 g (72 mmoles) d'oxyde de manganese et le milieu est agité à température ambiante une nuit. Le mélange est filtré sur célite et le solvant évaporé. On obtient 11,8 g (85%) du produit attendu sous la forme d'une huile.

### (c) 2-(3-Bromo-4-methyl-phenyl)-[1,3]dioxolane.

Dans un ballon et sous azote, on introduit 11,8 g (60 mmoles) de 3-Bromo-4-methyl-benzaldehyde dans 150 ml de toluène. 16,5 ml (300 mmoles) d'éthylène glycol et 571 mg (3 mmoles) d'acide paratoluènesulfonique sont rajoutés. On chauffe à reflux pendant 36 heures et sépare l'eau formée à l'aide d'un Dean-Stark. A température ambiante, le milieu réactionnel est versé dans une solution aqueuse saturée en sodium hydrogen carbonate puis extrait avec de l'éther. La phase organique est séchée sur sulfate de magnésium, filtrée et évaporée. On obtient 13 g (89%) du produit attendu sous la forme d'une huile marron.

### (d) Acide 5-[1,3]Dioxolan-2-y1-2-methyl-phény/boronique.

De manière analogue à l'exemple 2(k), par réaction de 11,1 g (46 moles) de 2-(3-Bromo-4-methyl-phenyl)-[1,3]dioxolane avec 12,3 ml (55 mmoles) de borate de triisopropyle, on obtient 6 g (88%) du produit sous la forme d'une huile.

### (e) 6-Methyl-4-propionyl-2'propyl-biphenyl-3-carbaldehyde.

De manière analogue à l'exemple 1(g), par réaction de 2 g (6,2 mmoles) de 1,1,1-Trifluoro-methanesulfonate de 4-propionyl-2-propyl-phenyle avec 1,2 g (8 mmoles) d'acide 5-[l13]Dioxolan-2-yl-2-methyl-phénylboronique, on obtient, après purification par chromatographie sur colonne de silice élué avec un mélange d'heptane et d'acétate d'éthyle (90/10), 1,42 g (78%) du produit sous la forme d'une huile jaune clair.

### (f) 4-Bromomethy/-phtha/ate de dimethyle.

Dans un ballon et sous courant d'azote, on introduit 10 g (44,6 mmoles) de 4-Hydroxymethyl-phthalate de diméthyle obtenu en 1(i) dans 75 ml de dichlorométhane. A 5°C, on rajoute une solution de 2,1 ml (22 mmoles) de phosphorus tribromide. 2 heures après, on ajoute lentement de l'eau. Après décantation, la phase aqueuse est extraite avec du dichlorométhane, les phases organiques sont réunies, séchées sur sulfate de magnésium, filtrées puis évaporées. On obtient 8,8 g (69%) du produit attendu sous la forme d'une huile jaune.

### (g) 4-(Diethoxy-phosphoMmethyl)-phthalate de dimethyle.

Dans un ballon, on introduit 65,7 g (229 mmoles) de 4-bromomethyl-phthalate de dimethyle dans 100 ml (583 mmoles) de triéthylphosphite. On chauffe au reflux pendant 4 heures. Le milieu est ensuite distillé pour éliminer la triéthylphosphite. On obtient 60,7 g (77%) du produit attendu sous la forme d'une huile jaune.

### (h) 4-[(Z)-2-(6-Methy/-4'-propiony/-2'-propyl-bipheny/-3-y/)-vinyl]-phthalate de dimethyle.

Dans un ballon et sous courant d'azote, on introduit 2 g (6 mmoles) de 4-(Diethoxyphosphorylmethyl)-phthalate de dimethyle dans 50 ml de THF. 3 ml de diisopropylamidure de lithium (2M/THF) sont ajoutés puis 1,42 g (5 mmoles) de 6-Methyl-4'-propionyl-2'-propyl-biphenyl-3-carbaldehyde. L'agitation est maintenue pendant 3 jours à température ambiante. Le milieu réactionnel est alors versé dans de l'eau et extrait à l'acétate d'éthyle. La phase organique est séchée sur sulfate de magnésium, filtrée puis évaporée. Le résidu obtenu est purifié par chromatographie sur colonne de silice élué avec un mélange d'heptane et d'acétate d'éthyle (90/10). Après évaporation des solvants, on obtient 900 mg (39%) du produit attendu sous la forme d'une huile jaune.

### (i) 4-{(Z)-2-[4'-(2-Ethyl-[1,3]dioxolan-2-yl)-6-methyl-2'-propyl-biphenyl-3-yl]-vinyl}-phthalate de dimethyle.

De manière analogue à l'exemple 6(c), à partir de 900 mg (1,9 mmoles) de 4-[(Z)-2-(6-Methyl-4'-propionyl-2'-propyl-biphenyl-3-yl)-vinyl]-phthalate de dimethyle, on obtient 700 mg (70%) du produit attendu sous la forme d'une huile jaune.

### (j) 4-{2-[4'-(2-Ethyl-[1,3]dioxolan-2-yl)-6-methyl-2'-propyl-biphenyl-3-yl]-ethyl}-phthalate de dimethyle.

Dans un réacteur, on introduit 700 mg (1,3 mmole) de 4-{(Z)-2-[4'-(2-Ethyl-[1,3]dioxolan-2-yl)-6-methyl-2'-propyl-biphenyl-3-yl]-vinyl}-phthalate de dimethyle dans 15 ml de méthanol, 15 ml d'acétate d'éthyle et 1 ml de triéthylamine. On dégaze le milieu réactionnel et on ajoute 100 mg de palladium sur charbon (10%) et hydrogène sous pression de 3,5 bars à 80°C. On filtre le milieu réactionnel, évapore et on obtient 640 mg (93%) du produit attendu sous la forme d'une huile.

### (k) (4-{2-[4'-(2-Ethyl-[1,3]dioxolan-2-yl)-6-methyl-2'-propyl-biphenyl-3-yl]-ethyl}-2-hydroxymethyl-phenyl)-methanol.

Dans un ballon et sous azote, on introduit 182 mg (4,8 mmoles) d'hydrure de lithiumaluminium dans 20 ml de THF sec. A 0°C, on ajoute, goutte à goutte, une solution de 640 mg (1,2 mmole) de 4-{2-[4'-(2-Ethyl-[1,3]dioxotan-2-yl)-6-methyl-2'-propyl-biphenyl-3-yl]-ethyl}-phthalate de dimethyle dans 5 ml de THF. Le milieu réactionnel est agité 1 heure à température ambiante. On ajoute, alors, très lentement, 200 µl d'eau, puis 200 µl d'une solution de soude à 15% puis 600 µl d'eau. Le milieu est filtré et le filtrat est évaporé à sec. On obtient 640 mg (100%) du produit attendu sous la forme d'une huile incolore.

### (1) 1-{5'-[2-(3,4-Bis-hydroxymethyl-pheny)-ethyl]-2'-methyl-2-propyl-biphenyl-4-yl}-propan-1-one.

Dans un ballon, on introduit 640 mg (1,4 mmole) de (4-{2-[4'-(2-Ethyl-[1,3]dioxolan-2-yl)-6-methyl-2'-propyl-biphenyl-3-yl]-ethyl}-2-hydroxymethyl-phenyl)-methanol dans 15 ml d'acétone et 15 ml d'eau. Une pointe de spatule d'acide p-toluènesulfonique est rajoutée et la solution est portée au reflux pendant 2 heures 30. A température ambiante, elle est versée dans une solution aqueuse saturée en sodium hydrogen carbonate et extraite à l'acétate d'éthyle. La phase organique est séchée sur sulfate de magnésium, filtrée puis évaporée. Le résidu obtenu est purifié par chromatographie sur colonne de silice élué avec un mélange d'heptane et d'acétate d'éthyle (30/70). On obtient 300 mg (50%) du produit attendu sous la forme d'une huile incolore.

### (m) (4-{2-[4'-(1-Ethyl-1-hydroxy-propyl)-6-methyl-2'-propyl-biphenyl-3-yl]-ethyl}-2-hydroxymethyl-phenyl)-methanol.

De manière analogue à l'exemple 1(q), par réaction de 300 mg (0,7 mmoles) de 1-{5'-[2-(3,4-Bis-hydroxymethyl-phenyl)-ethyl]-2'-methyl-2-propyl-biphenyl-4-yl}-propan-1-one avec 1,9 ml (5,6 mmoles) de bromure d'éthylmagnésium (3M/éther), on obtient, après purification sur colonne de silice élué avec un mélange d'heptane et d'acétate d'éthyle (40/60). 180 mg (56%) de (4-{2-[4'-(1-Ethyl-1-hydroxy-propyl)-6-methyl-2'-propyl-biphenyl-3-yl]-ethyl}-2-hydroxymethyl-phenyl)-methanol sous la forme de cristaux blancs de point de fusion 76°C.

### EXEMPLE 6 : (4-{[4'-(1-Ethyl-1-hydroxy-propyl)-6.2'-dimethyl-biphenyl-3-ylamino]-methyl}-2-hydroxymethyl-phenyl)-methanol.

### (a) 1-(4-Bromo-3-methyl-phenyl)-propan-1-one.

24,7 g (126 mmol) de 4-bromo-3-méthyl-benzonitrile sont dissous dans 400 mL de dioxanne anhydre, puis le milieu est refroidi à 0°C. 124 mL (372 mmol) de bromure d'éthylmagnésium sont additionnés goutte à goutte, puis le milieu est ramené à température ambiante et agité pendant 4 heures. Le milieu réactionnel est traité par 250 mL de HCl 3N, puis extrait par un mélange eau / éther. Un solide blanc est obtenu après trituration dans l'heptane (m = 14,5 g, R = 52%).

### (b) 2-(4-Bromo-3-methyl-phenyl)-2-ethyl-[1,3]dioxolane.

13 g (57 mmol) de 1-(4-bromo-3-methyl-phenyl)-propan-1-one sont dissous dans 130 mL de toluène. 54 mL (800 mmol) d'éthylène glycol sont ajoutés, puis 1,4 g (7,4 mmol) d'acide para-toluène sulfonique. Le milieu est équipé avec un montage de distillation de type Dean-Stark et chauffé à 125°C pendant 14 heures. Après traitement avec une solution de soude diluée, la phase organique est lavée à l'eau et concentrée sous pression réduite. Une huile jaune est obtenue (m =13,6 g, R = 88%).

### (c) 2-Methyl-4-propionyl-benzeneboronic acid.

1,6 g (65 mmol) de magnésium sont suspendus dans 5 mL de THF et 0,5 mL de 1,2-dibromoéthane. Un cristal d'iode est ajouté. Une solution de 13,6 g (50 mmol) de 2-(4-bromo-3-methyl-phenyl)-2-ethyl-[1,3]dioxolane et 0,4 ml de 1,2-dibromoéthane dans 70 mL de THF est additionnée lentement. Le milieu est maintenu à reflux pendant 45 minutes après la fin de l'addition, puis refroidi à -78°C. 6,2 mL (55 mmol) de triméthyl borate sont ajoutés, et le milieu est maintenu à cette température pendant 45 minutes. 100 mL de HCL 1N sont alors additionnés, et le milieu est ramené à température ambiante. Après décantation et concentration de la phase, organique, le résidu obtenu est trituré avec de l'heptane: un solide beige est obtenu (m = 5,1 g, R = 54%).

### (d) 1-(2,2'-Dimethyl-5'-nitro-biphenyl-4-yl)-propan-1-one.

Dans un ballon, on introduit 2,5 g (13 mmol) de 2-methyl-4-propionyl-benzeneboronic acid dans 100 ml de diméthoxyéthane et on ajoute 2,2 g (10 mmol) de 2-bromo-4-nitrotoluène et 13 mL d'une solution aqueuse 2M de carbonate de potassium. Le milieu est dégazé pendant 10 minutes par un flux d'azote, puis 580 mg (0,5 mmol) de tetrakistriphenylphosphine palladium. Le milieu est agité pendant 14 heures à 90°C, puis soumis au traitement usuel. Le résidu est obtenu après chromatographie sur colonne de silice (éluant acétate d'éthyle 1 / heptane 9) et on obtient 2,3 g (81%) du produit attendu sous la forme d'une huile jaune.

### (e) 1-(5'-Amino-2,2'-dimethyl-biphenyl)-4-yl)-propan-1-one.

2,2 g (7,8 mmol) de 1-(2,2'-dimethyl-5'-nitro-biphenyl-4-yl)-propan-1-one sont dissous dans un mélange de 80 mL d'éthanol et 40 mL d'eau et 10 mL d'acide acétique. Le mélange est chauffé à reflux alors que 1,7 g (31 mmol) de poudre fer sont ajoutés. Le milieu est chauffé pendant 1 h, puis traité par addition d'ammoniaque, refroidi et filtré sur de la célite. La solution obtenue est diluée dans de l'acétate d'éthyle, puis lavée à l'eau, et la phase organique est séchée et concentrée sous pression réduite. Après chromatographie sur colonne de silice (éluant heptane 60 / acétate d'éthyle 40), une huile jaune est obtenue (m =1,9 g ; R = 96%).

### (f) (6,2'-Dimethyl-4'-propionyl-biphenyl-3-yl)-carbamate de tert-butyle.

800 mg (3,15 mmol) de 1-(5'-amino-2,2'-dimethyl-biphenyl-4-yl)-propan-1-one sont dissous dans 50 mL de dichlorométhane. 500 µL (3,5 mmol) de triéthylamine sont ajoutés, suivis de 760 mg (3,5 mmol) de dicarbonate de di-tert-butyle. Le milieu est agité à température ambiante pendant 12 heures, puis 2 heures à 50°C. Le milieu est traité avec de l'eau, puis le résidu est purifié par chromatographie sur colonne de silice (éluant heptane 3 / acétate d'éthyle 1), pour obtenir un solide cristallin jaune (pf 119°C, m = 1,05 g, R = 94%).

### (g) Benzoate de 2-benzoyloxymethyl-5-{[tert-butoxycarbonyl-(6,2'-dimethyl-4'-propionylbiphenyl-3-yl)-amino]-methyl}-benzyle.

900 mg (2,5 mmol) de (6,2'-dimethyf-4'-propionyl-biphenyl-3-yl)-carbamate de *tert-*butyle sont dissous dans 30 mL de diméthylformamide. 110 mg (2,8 mmol) d'hydrure de sodium 60% sont ajoutés, et le milieu est agité pendant 15 minutes. 1,23 g (2,8 mmol) de bromure de (3,4-bis-benzoyloxyméthyl)benzyle sont alors additionnés, et le milieu est agité pendant 12 heures à température ambiante. Après un traitement usuel, le résidu obtenu est purifié par chromatographie sur colonne, de silice (éluant heptane 8 / acétate d'éthyle 2). Une huile incolore est obtenue (m =1 g, R = 55%).

### (h) Benzoate de 2-benzoyloxymethyl-5-[(6,2'-dimethyl-4'-propionyl-biphenyl-3-ylamino)-methyl]-benzyle.

1 g (1,4 mmol) de benzoate de 2-benzoyloxymethyl-5-{[*tert*-butoxycarbonyl-(6,2'-dimethyl-4'-propionyl-biphenyh3-yl)-amino]-methyl}-benzyle sont dissous dans 50 mL de dichlorométhane. 1,1 mL (14 mmol) d'acide trifluoroacétique sont ajoutés, et le milieu est agité à température ambiante pendant 6 heures. Après extraction eau-dichlorométhane, le produit désiré est obtenu sous forme d'huile jaunâtre, (m= 820 mg , R = 95%).

### (i) 1-[5'-(3,4-Bis-hydroxymethyl-benzylamino)-2,2-dimethyl-biphenyl-4-yl]-propan-1-one.

800 mg (1,3 mmol) de benzoate de 2-benzoyloxymethyl-5-[(6,2'-dimethyl-4'-propionylbiphenyl-3-ylamino)-methyl]-benzyle sont dissous dans une solution méthanolique de carbonate de potassium à 2%, et le milieu est agité pendant 1 heure. Après extraction eau / dichlorométhane; le résidu obtenu est purifié par chromatographie sur gel de silice (éluant heptane 30 / acétate d'éthyle 70). Une huile jaune est obtenue (m = 470 mg, R = 88%).

### (j) (4-{[4'-(1-Ethyl-1-hydroxy-propyl)-6,2'-dimethyl-biphenyl-3-ylamino]-methyl}-2-hydroxymethyl-phenyl)-methanol.

450 mg (1,1 mmol) de 1-[5'-(3,4-bis-hydroxymethyl-benzylamino)-2,2'-dimethyl-biphenyl-4-yl]-propan-1-one sont dissous dans 30 mL de THF. 1,5 mL (4,5 mmol) de bromure d'ethylmagnesium sont additionnés, et le milieu est agité à température ambiante pendant 1 heure. Après le traitement usuel, le résidu est purifié par chromatographie sur gel de silice (éluant heptane 1 / acétate d'éthyle 1). Un solide blanc est obtenu (m = 130 mg ; R = 27%).
RMN ¹H (CDCl₃): 0,80 (t, 6H, J = 7,4 Hz); 1,85 (q, 4H, J = 7,5 Hz); 1,91 (s, 3H); 2,06 (s, 3H); 2,86 (bs, 2H); 4,30 (s, 2H); 4,72 (s, 2H); 4,73 (s, 2H); 6,45 (d, 1H. J = 2,4 Hz); 6,55 (dd, 1H. J1 = 2,4 Hz, J2 = 8,1 Hz); 7,04 (m, 2H); 7,15 (m, 1H); 7,24-7,37 (m, 4H).

### EXEMPLE 7 : FORMULATIONS

### 1) VOIE ORALE

(a) On prépare la composition suivante sous la forme d'un comprimé de 0,2 g

| | |
|---|---|
| Composé de l'exemple 2 | 0,005 g |
| Amidon prégélatinisé | 0,065 g |
| Cellulose microcristalline | 0,075 g |
| Lactose | 0,050 g |
| Stéarate de magnésium | 0,005 g |

Pour le traitement de l'ichtyose, on administre à un individu adulte 1 à 3 comprimés par jour pendant 1 à 12 mois selon la gravité du cas traité.
(b) On prépare une suspension buvable, destinée à être conditionnée en ampoules de 5 ml

| | |
|---|---|
| Composé de l'exemple 6 | 0,050 mg |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p | 5 ml |

Pour le traitement de l'acné, on administre à un individu adulte 1 ampoule par jour pendant 1 à 12 mois selon la gravité du cas traité.
(c) On prépare la formulation suivante destinée à être conditionnée en gélules :

| | |
|---|---|
| Composé de l'exemple 5 | 0,0001 mg |
| Amidon de maïs | 0,060 g |
| Lactose q.s.p | 0,300 g |

Les gélules utilisées sont constituées de gélatine, d'oxyde de titane et d'un conservateur.
Dans le traitement du psoriasis, on administre à un individu adulte 1 gélule par jour pendant 1 à 12 mois.
(d) On prépare la formulation suivante destinée à être conditionnée en gélules :

| | |
|---|---|
| Composé de l'exemple 1 | 0,02 mg |
| Cyclosporine | 0,050 g |
| Amidon de maïs | 0,060 g |
| Lactose q.s.p | 0,300 g |

Les gélules utilisées sont constituées de gélatine, d'oxyde de titane et d'un conservateur.
Dans le traitement du psoriasis, on administre à un individu adulte, 1. gélule par jour pendant 1 à 12 mois.

### 2) VOIE TOPIQUE

(a) On prépare la crème Eau-dans l'Huile non ionique suivante :

| | |
|---|---|
| Composé de l'exemple 5 | 0,100 g |
| Mélange d'alcools de lanoline émulsifs, de cires et d'huiles raffinés, vendu par la Société Beiersdorf sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 1 à 12 mois.
(b) On prépare un gel en réalisant la formulation suivante :

| | |
|---|---|
| Composé de l'exemple 2 | 0,001 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 1 | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 3 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition est appliquée quotidiennement, elle permet de lutter contre le vieillissement photoinduit.
(e) On prépare la crème Huile dans l'Eau suivante :

| | |
|---|---|
| Composé de l'exemple 5 | 0,500 g |
| Acide rétinoique | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 jours pour traitement d'attaque et indéfiniment pour entretien.
(f) On prépare un gel topique en procédant au mélange des ingrédients suivants :

| | |
|---|---|
| Composé de l'exemple 2 | 0,050 g |
| Ethanol | 43,000 g |
| α-tocophérol | 0,0509 |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100.000 g |

Ce gel est appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 4 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytotuène | 0,02 g |
| Eau qsp | 100,00 g |

On applique cette lotion 1 à 2 fois par jour pendant 3 mois sur un cuir chevelu ayant subi une chute de cheveu et indéfiniment pour traitement d'entretien.
(h) On prépare une crème anti-acnéique en procédant au mélange des ingrédient suivants :

| | |
|---|---|
| Composé de l'exemple 6 | 0,050 g |
| Acide rétinoique | 0,010 g |
| Mélange de stéarates de glycérol et de polyéthylène glycol (75 moles) vendu sous le nom de "Gelot 64" par ta société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

Cette crème est appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 2 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthyléne à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualéne | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000 g |

Cette crème est appliquée 2 fois par jour sur une peau atteinte de dermatose inflammatoire pendant 30 jours.
(j) On prépare la crème de type huile dans l'eau suivante :

| | |
|---|---|
| Acide lactique | 5,000 g |
| Composé de l'exemple 1 | 0,020g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthyléné à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydmsqualéne | 18,000 g |
| Mélange de triglycérides caprylique-caprique vendu sous la dénomination de "Miglyol 812" par la société "DYNAMIT NOBEL" | 4,000 g |
| Eau q.s.p. | 100,000 g |

Cette crème est appliquée 1 fois par jour, elle aide à lutter contre le vieillissement qu'il soit photoinduit ou chronologique.
(k) On prépare l'onguent anhydre suivant :

| | |
|---|---|
| Composé de l'exemple 5 | 5,000 g |
| Huile de vaseline | 50,00 g |
| Butylhydrotoluène | 0,050 g |
| Vaseline blanche | qs 100 g |

Cet onguent est appliqué 2 fois par jour sur une peau atteinte de dermatose squameuse pendant 30 jours.

### 3) VOIE INTRALESIONNELLE

(a) On prépare la composition suivante :

| | |
|---|---|
| Composé de l'exemple 1 | 0,002 g |
| Oléate d'éthyle | qs 10 g |

Dans le traitement du mélanome malin, on injecte la composition à un individu adulte à une fréquence de 1 à 7 fois par semaine pendant 1 à 12 mois.
(b) On prépare la composition suivante :

| | |
|---|---|
| Composé de l'exemple | 20,050 g |
| Huile d'olive | qs 2 g |

Dans le traitement du carcinome basocellulaire, on injecte la composition à un individu adulte à une fréquence de 1 à 7 fois par semaine pendant 1 à 12 mois.
(c) On prépare la composition suivante :

| | |
|---|---|
| Composé de l'exemple 3 | 0,1 mg |
| Huile de sésame | qs 2 g |

Dans le traitement du carcinome spinocellulaire, on injecte la composition à un individu adulte à une fréquence de 1 à 7 fois par semaine pendant 1 à 12 mois.
(d) On prépare la composition suivante :

| | |
|---|---|
| Composé de l'exemple 4 | 0,001 mg |
| Benzoate de méthyle | qs 10 g |

Dans le traitement du carcinome du colon, on injecte la composition à un individu adulte à une fréquence de 1 à 7 fois par semaine pendant 1 à 12 mois.

### 4) VOIE INTRAVEINEUSE

(a) On prépare l'émulsion lipidique injectable suivante :

| | |
|---|---|
| Composé de l'exemple 5 | 0,001mg |
| Huile de soja | 10,000 g |
| Phospholipide d'oeuf | 1,200 g |
| Glycérine | 2,500 g |
| Eau pour injectable q.s.p. | 100,000 g |

Dans le traitement du psoriasis, on injecte la composition à un individu adulte à une fréquence de 1 à 7 fois par semaine pendant 1 à 12 mois.
(b) On prépare l'émulsion lipidique injectable suivante :

| | |
|---|---|
| Composé de l'exemple 6 | 0,010 g |
| Huile de coton | 10,000 g |
| Lécithine de soja | 0,750 g |
| Sorbitol | 5,000 g |
| DL,a Tocophérol | 0,100 g |
| Eau pour injectable q.s.p. | 100,000 g |

Dans le traitement de l'ichtyose, on injecte la composition à un individu adulte à une fréquence de 1 à 7 fois par semaine pendant 1 à 12 mois.
(c) On prépare l'émulsion lipidique injectable suivante :

| | |
|---|---|
| Composé de l'exemple | 0,001 g |
| Huile de soja | 15,000 g |
| Monoglycérides acétylés | 10,000 g |
| Pluronic F-108 | 1,000 g |
| Glycérol | 2,500 g |
| Eau pour injectable q.s.p. | 100,000 g |

Dans le traitement de la leucémie, on injecte la composition à un individu adulte à une fréquence de 1 à 7 fois par semaine pendant 1 à 12 mois.
(d) On prépare la composition micelle mixte suivante :

| | |
|---|---|
| Composé de l'exemple 2 | 0,001 g |
| Lécithine | 16,930 g |
| Acide glycocholique | 8,850 g |
| Eau pour injectable q.s.p. | 100,000 g |

Dans le traitement du mélanome malin, on injecte la composition à un individu adulte à une fréquence de 1 à 7 fois par semaine pendant 1 à 12 mois.
(e) On prépare la composition de cyclodextrine suivante :

| | |
|---|---|
| Composé de l'exemple 5 | 0,1mg |
| βcyclodextrine | 0,100 g |
| Eau pour injectable q.s.p. | 10,000 g |

Dans le traitement du rejet de greffe, on injecte la composition à un individu adulte à une fréquence de 1 à 7 fois par semaine pendant 1 à 12 mois.
(f) On prépare la composition de cyclodextrine suivante :

| | |
|---|---|
| Composé de l'exemple 3 | 0,010 g |
| 2-hydroxypropylficydodextrine | 0,100 g |
| Eau pour injectable q.s.p. | 10,000 g |

Dans le traitement du cancer du rein, on injecte la composition à un individu adulte à une fréquence de 1 à 7 fois par semaine pendant 1 à 12 mois.

### EXEMPLE 8: Tests d'évaluation de l'activité biologiques des composés de l'invention - Activité sur la différenciation des cellules HL60

Le calcitriol induit la différenciation des cellules de leucémie promyelocytaire (HL60) en monocytes/macrophages. Cet effet inducteur de différenciation est un marqueur bien caractérisé de la vitamine D cellulaire. Un des produits antimicrobiens le plus important des macrophages, est le peroxyde d'hydrogène, qui peut être analysé expérimentalement par la réduction du NBT (Nitroblue Tetrazolium).
La méthode utilisée est la suivante : les cellules HL60 sont ensemencées dans des plaques 6 puits puis traitées immédiatement avec un composé à tester. Après 4 jours de culture, les cellules sont incubées avec du TPA ester de phorbol et le NBT pendant une courte période et les cellules différenciés, c'est-à-dire, positives au NBT dont comptabilisées.
L'effet inducteur de la différenciation sur les cellules HL60 des composés selon invention ainsi que celui du composé de référence, le calcitriol, figure dans le tableau I.
Les résultats montrent que les composés des exemptes 1 à 4 ont une activité d'induction de la différenciation sur les cellules HL60 similaire à celle du calcitriol, le composé de l'exemple 5 ayant même une activité nettement supérieure.

Quant au composé de l'exemple 6, il présente une moins bonne activité comparée au calcitriol mais reste néanmoins un composé très intéressant comparé aux composés de l'état de la technique.

**Tableau I**

| *Composé testé* | *AC50-HL60 (en nM)* |
|---|---|
| Calcitriol | 10,7 |
| Composé de l'exemple 1 | 7,6 |
| Composé de l'exemple 2 | 15,0 |
| Composé de l'exemple 3 | 11,0 |
| Composé de l'exemple 4 | 27,0 |
| Composé de l'exemple 5 | 1,8 |
| Composé de l'exemple 6 | 147 |

### EXEMPLE 9: Tests d'évaluation de l'activité biologique des composés de l'invention - Mesure de l'activité agoniste VDR (AC50 hVDR)

L'activité agoniste VDR des composés de l'invention peut être testée sur la lignée cellulaire HeLa par cotransfection du vecteur d'expression du récepteur VDR humain et du plasmide rapporteur p240Hase-CAT qui contient la région -1399 à +76 du promoteur de la 24-hydroxylase de rat, clonée en amont de la phase codante du gène de chlorampénicol-acétyl-transférase (CAT). 18 heures après cotransfection, le composé à tester est ajouté dans le milieu. Après 18 heures de traitement, le dosage de l'activité CAT des lysats cellulaires est effectué par un test ELISA (Enzyme Linked Immuno Sorbent Essay, commercialisé par Roche Molecular Biochemicals). L'activité agoniste peut être caractérisée dans ce système de cotransfection par la détermination de la dose nécessaire pour atteindre 50% de l'activité maximale du composé testé (AC50).
Le mesure de l'activité agoniste VDR des composés selon l'invention ainsi que celui du composé de référence, le calcitriol, figure dans le tableau II.
Ces résultats montrent que les composés selon la présente invention ont des activités comparables à celle du calcitriol, le composé de l'exemple 5 ayant, là encore, une activité supérieure.

Ces résultats montrent là aussi que le composé de l'exemple 6 présente une moins bonne activité comparée au calcitriol, il reste néanmoins un composé très intéressant comparé aux composés de l'état de la technique.

**Tableau II**

| *Composé testé* | *AC50-hVOR (en nM)* |
|---|---|
| Calcitriol | 2,5 |
| Composé de l'exemple 1 | 2,4 |
| Composé de l'exemple 2 | 4,1 |
| Composé de l'exemple 3 | 2,2 |
| Composé de l'exemple 4 | 3,2 |
| Composé de l'exemple 5 | 0,6 |
| Composé de l'exemple 6 | 50 |

### EXEMPLE 10: Tests d'évaluation de l'activité biologique des composés de l'invention - Activité sur la prolifération des kératinocytes humains

Il est connu que la 1,25-dihydroxyvitamine D3, appelée calcitriol et correspondant à la vitamine D naturelle inhibe la prolifération des kératinocytes humains en culture.
La méthode utilisée est la suivante: les kératinocytes humains normaux sont ensemencés à basse densité dans une plaque 24 puits. Après 4 heures, les composés à tester sont ajoutés au milieu de culture. Après 5 jours de culture, la prolifération des kératinocytes est déterminée par incorporation de 5-bromo-2' deoxyuridine (BrdU) dans l'ADN. La quantitée de BrdU incorporée est ensuite mesurée en utilisant le test ELISA (Enzyme Linked Immuno Sorbent Essay, commercialisé par Roche Molecular Biochemicals).
L'effet inhibiteur sur la prolifération des kératinocytes des composés selon l'invention et du calcitriol utilisé comme composé de référence est résumé dans le tableau III.
La valeur IC50 indique la concentration du composé testé pour laquelle le composé inhibe de 50 % la prolifération des kératinocytes.
Ces résultats permettent de montrer que les composés de l'invention ont une activité inhibitrice sur la prolifération des kératinocytes dans les mêmes gammes de valeur que le calcitriol, le composé de l'exemple 5 se démarque par une activité plus de 5 fois supérieure à celle du calcitriol.

Quant au composé de l'exemple 6, il présente une moins bonne activité comparée au calcitriol mais reste néanmoins un composé très intéressant comparé aux composés de l'état de la technique.

**Tableau III**

| *Activité mesurée* | *IC50 - proliférations des KHN (en nM)* |
|---|---|
| Calcitriol | 15,3 |
| composé de l'exemple 1 | 16,0 |
| Composé de l'exemple 2 | 53,0 |
| Composé de l'exemple 3 | 15,0 |
| Composé de l'exemple 4 | 26,0 |
| Composé de l'exemple 5 | 2,4 |
| Composé de l'exemple 6 | 122 |

## Revendications

1. Composés, **caractérisés par le fait qu'**ils sont pris, seuls ou en mélanges, dans le groupe constitué par :
1-{5-[4'-(1-Ethyl-1-hydroxypropyl)-6-methyl-2'-propylbiphenyl-3-yloxymethyl]-2-hydroxymethylphenyl}methanol;
2-{5-[6,2'-Diethyl-4'-(1-ethyl-1-hydroxypropyl)biphenyl-3-yloxymethyl]-2-hydroxymethylphenyl}methanol;
3-{4-[6-Ethyl-4'-(1-ethyl-1-hydroxypropyl)-2'-propylbiphenyl-3-yloxymethyl]-2-hydroxymethylphenyl}methanol;
4-{4-[6-Ethyl-4'-(1-ethyl-1-hydroxypropyl)-2'-isopropylbiphenyl-3-yloxymethyl]-2-hydroxymethylphenyl}methanol;
5-(4-{2-[4'-(1-Ethyl-1-hydroxypropyl)-6-methyl-2'-propylbiphenyl-3-yl]ethyl}-2-hydroxymethylphenyl)methanol;
6-{4-[4'-(1-Ethyl-1-hydroxypropyl)-6-methyl-2'-propylbiphenyl-3-ylmethoxy]-2-hydroxymethylphenyl}methanol;
7-(4-{[4'-(1-Ethl-1-hydroxypropyl)-6-methyl-2'-propylbiphenyl-3-ylamino]methyl}-2-hydroxymethylphenyl)methanol;
8-[4-({[4'-(1-Ethyl-1-hydroxypropyl)-6-methyl-2'-propylbiphenyl-3-yl]methylamino}methyl)2-hydroxymethylphenyl]methanol;
9-[4-({Ethyl-[4'-(1-ethyl-1-hydroxypropyl)-6-methyl-2'-propylbiphenyl-3-yl]amino}methyl)-2-hydroxymethylphenyl]methanol;
10-[4-({[4'-(1-Ethyl-1-hydroxypropyl)-6-methyl-2'-propylbiphenyl-3-yl]propylamino}methyl)-2-hydroxymethylphenyl]methanol;
11-(2-Hydroxymethyl-4-{2-[6-methyl-2'-propyl-4'-(2,2,2-trifluoro-1-hydroxy-1trifluoromethyl-ethyl)biphenyl-3-yl]ethyl}phenyl)methanol;
12-{2-Hydroxymethyl-4-[6-methyl-2'-propyl-4'-(2,2,2-trifluoro-1-hydroxy-1-trifluoromethylethyl)biphenyl-3-yloxymethyl]phenyl}methanol;
13- {2-Hydroxymethyl-4-[6-methyl-2'-propyl-4'-(2,2,2-trifluoro-1-hydroxy-1-trifluoromethylethyl)biphenyl-3-ylmethoxy]phenyl}methanol;
14-(2-Hydroxymethyl-4-{[6-methyl-2'-propyl-4'-(2,2,2-trifluoro-1-hydroxy-1-trifluoromethylethyl)biphenyl-3-ylamino]methyl}phenyl)methanol;
15-[2-Hydroxymethyt-4-({N-methyl[6-methyl-2'-propyl-4'-(2,2,2-trifluoro-1-hydroxy-1-trifluoromethyl-ethyl)biphenyl-3-yl]amino}methyl)phenyl]methanol;
16-[4-({N-Ethyl[6-methyl-2'-propyl-4'-(2.2,2-trifluoro-1-hydroxy-1-trifluoromethylethyl)biphenyl-3-yl]amino}methyl)-2-hydroxymethylphenyl]methanol;
17-[2-Hydroxymethyl-4-({[6-methyl-2'-propyl-4'-(2,2,2-trifluoro-1-hydroxy-1-trifluoromethyl-ethyl)biphenyl-3-yl]N-propyl-amino}methyl)phenyl]methanol;
18-(4-{2-[6-Ethyl-4'-(1-ethyl-1-hydroxypropyl)-2'-propylbiphenyl-3-yl]ethyl}-2-hydroxymethylphenyl)methanol;
19-{4-[6-Ethyl-4'-(1-ethyl-1-hydroxypropyl)-2'-propylbiphenyl-3-ylmethoxy]-2-hydroxymethylphenyl}methanol;
20-(4-{[6Ethyl-4'-(1-ethyl-1-hydroxypropyl)-2'-propylbiphenyl-3-ylamino]methyl}-2-hydroxymethylphenyl)methanol;
21-[4-({[6-Ethyl-4'-(1-ethyl-1-hydroxypropyl)-2'-propylbiphenyl-3-yl]methylamino}methyl)-2-hydroxymethylphenyl]methanol;
22-[4-({Ethyl-[6-ethyl-4'-(1-ethyl-1-hydroxypropyl)-2'-propylbiphenyl-3-yl]amino}methyl)-2-hydroxymethylphenyl]methanol;
23-[4-({[6-Ethyl-4'-(1-ethyl-1-hydroxypropyl)-2'-propylbiphenyl-3-yl]propylamino}methyl)-2-hydroxymethytphenyl]methanol;
24-(4-{2-[6-Ethyl-2'-propyl-4'-(2,2,2-trifluoro-1-hydroxy-1-trifluoromethyl-ethyl)biphenyl-3-yl]ethyl}-2-hydroxymethylphenyl)methanol;
25-{4-[6-Ethyl-2'-propyl-4'-(2,2,2-trifluoro-1-hydroxy-1-trifluoromethyl-ethyl)biphenyl-3-yloxymethyl]-2-hydroxymethylphenyl}methanol;
26-{4-[6Ethyl-2'-propyl-4'-(2,2.2-trifluoro-1-hydroxy-1-trifluoromethyl-ethyl)biphenyl-3-ylmethoxy]-2-hydroxymethylphenyl}methanol;
27-(4-{[6-Ethyt-2'-propyl-4'-(2.2.2-trifluoro-1-hydroxy-1-trifluoromethyl-ethyl)biphenyl-3-ylamino]methyl}-2-hydroxymethylphenyl)methanol;
28-[4-({[6-Ethyl-2'-propyl-4'-(2,2,2-trifiuoro-1-hydroxy-1-trifluoromethyl-ethyl)biphenyl-3-yl]methylamino}methyl)-2-hydroxymethylphenyl]methanol;
29-[4-({N-Ethyl[6-ethyl-2'-propyl-4'-(2,2,2-trifluoro-1-hydroxy-1-trifluoromethylethyl)biphenyl-3-yl]amino}methyl)-2-hydroxymethylphenyl]methanol;
30-[4-({[6-Ethyl-2'-propyl-4'-(2,2,2-trifluoro-1-hydroxy-1-trifluoromethyl-ethyl)biphenyl-3-yl]-N-propyl-amino}methyl)-2-hydroxymethylphenyl]methanol;
31-(4-{[4'-(1-Ethyl-1-hydroxypropyl)-6.2'-dimethylbiphenyl-3-ylamino]methyl}-2-hydroxymethylphenyl)methanol.

2. Composés selon la revendication 1 à titre de médicament.

3. Utilisation d'un ou plusieurs composés selon la revendication 1 pour la fabrication d'une composition pharmaceutique destinée au traitement :
1- des affections dermatologiques liées à un désordre de la différenciation ou de la prolifération des kératinocytes ou des sébocytes ;
2- des troubles de la kératinisation ;
3- des affections dermatologiques liées à un trouble de la kératinisation avec une composante inflammatoire et/ou immuno-allergique ;
4- des affections inflammatoires ne présentant pas de trouble de la kératinisation ;
5- des proliférations dermiques ou épidermiques ;
6- des désordres dermatologiques tels que les dermatoses bulleuses et les maladies du collagène;
7- du vieillissement de la peau, qu'il soit photo-induit ou chronologique ou pour réduire les pigmentations et les kératoses actiniques, ou toutes pathologies cutanées associées au vieillissement chronologique ou actinique ;
8- des troubles de la cicatrisation et des vergetures ;
9- des troubles de la fonction sébacée tels que l'hyperséborrhée de l'acné ou la séborrhée simple ou encore l'eczéma séborrhéique ;
10- des affections dermatologiques à composante immunologique.

4. Utilisation selon la revendication 3, **caractérisée en ce que** les affections dermatologiques liées à un désordre de la différenciation ou de la prolifération des kératinocytes ou des sébocytes concernent les acnés vulgaires, comédoniennes, polymorphes, rosacées, les acnés nodulokystiques, conglobata, les acnés séniles, les acnés secondaires telles que l'acné solaire, médicamenteuse ou professionnelle.

5. Utilisation selon la revendication 3, **caractérisée en ce que** les troubles de la kératinisation concernent les ichtyoses, les états ichtyosiformes, la maladie de Darier, les kératodermies palmoplantaires, les leucoplasies et les états leucoplasiformes, le lichen cutané ou muqueux (buccal).

6. Utilisation selon la revendication 3, **caractérisée en ce que** les affections dermatologiques liées à un trouble de la kératinisation avec une composante inflammatoire et/ou immuno-allergique concernent toutes les formes de psoriasis qu'il soit cutané, muqueux ou unguéal, le rhumatisme psoriatique, et l'atopie cutanée, telle que l'eczéma ou l'atopie respiratoire ou l'hypertrophie gingival.

7. Utilisation selon la revendication 3, **caractérisée en ce que** les proliférations dermiques ou épidermiques peuvent être bénignes ou malignes, d'origine non virale ou d'origine virale telles que verrues vulgaires, les verrues planes et l'épidermodysplasie verruciforme, les papillomatoses orales ou florides et **en ce que** les proliférations pouvent être induites par les ultra-violets notamment dans le cas des épithélioma baso et spinocellulaires.

8. Composition pharmaceutique, **caractérisée par le fait qu'**elle comprend, dans un support pharmaceutiquement acceptable, au moins l'un des composés tels que définis à la revendication 1.

9. Composition selon la revendication 8,**caractérisée en ce que** la concentration en composé(s) selon la revendication 1 est comprise entre 0,001 % et 5 % en poids par rapport au poids total de la composition.

10. Composition cosmétique, **caractérisée par le fait qu'**elle comprend, dans un support cosmétiquement acceptable, au moins l'un des composés tels que définis à la revendication 1.

11. Composition selon la revendication 10, **caractérisée en ce que** la concentration en composé(s) est comprise entre 0,001 % et 3 % en poids par rapport au poids total de la composition.

12. Utilisation cosmétique d'une composition cosmétique telle que définie à l'une des revendications 10 ou 11 pour l'hygiène corporelle ou capillaire.

13. Utilisation cosmétique d'une composition cosmétique telle que définie à l'une des revendications 10 ou 11 pour prévenir et/ou traiter le vieillissement cutané photo-induit ou chronologique.

## Claims

1. Compounds, **characterized in that** they are taken, alone or as mixtures, from the group consisting of:
1-{5-[4'-(1-Ethyl-1-hydroxypropyl)-6-methyl-2'-propylbiphenyl-3-yloxymethyl]-2-hydroxymethylphenyl}methanol;
2-{5-[6,2'-Diethyl-4'-(1-ethyl-1-hydroxypropyl)biphenyl-3-yloxymethyl]-2-hydroxymethylphenyl}methanol;
3-{4-[6-Ethyl-4'-(1-ethyl-1-hydroxypropyl)-2'-propylbiphenyl-3-yloxymethyl]-2-hydroxymethylphenyl}methanol;
4-{4-[6-Ethyl-4'-(1-ethyl-1-hydroxypropyl)-2'-isopropylbiphenyl-3-yloxymethyl]-2-hydroxymethylphenyl}methanol;
5-(4-{2-[4'-(1-Ethyl-1-hydroxypropyl)-6-methyl-2'-propylbiphenyl-3-yl]ethyl}-2-hydroxymethylphenyl)methanol;
6-{4-[4'-(1-Ethyl-1-hydroxypropyl)-6-methyl-2'-propylbiphenyl-3-ylmethoxy]-2-hydroxymethylphenyl}methanol;
7-(4-{[4'-(1-Ethyl-1-hydroxypropyl)-6-methyl-2'-propylbiphenyl-3-ylamino]methyl}-2-hydroxymethylphenyl)methanol;
8-[4-({ [4'-(1-Ethyl-1-hydroxypropyl)-6-methyl-2'-propylbiphenyl-3-yl]methylamino}methyl)-2-hydroxymethylphenyl]methanol;
9-[4-({Ethyl- [4'-(1-ethyl-1-hydroxypropyl)-6-methyl-2'-propylbiphenyl-3-yl]amino}methyl)-2-hydroxymethylphenyl]methanol;
10-[4-({ [4'-(1-Ethyl-1-hydroxypropyl)-6-methyl-2'-propylbiphenyl-3-yl]propylamino}methyl)-2-hydroxymethylphenyl]methanol;
11-(2-Hydroxymethyl-4-{2-[6-methyl-2'-propyl-4'-(2,2,2-trifluoro-1-hydroxy-1-trifluoromethylethyl)biphenyl-3-yl]ethyl}phenyl)methanol;
12-{2-Hydroxymethyl-4-[6-methyl-2'-propyl-4'-(2,2,2-trifluoro-1-hydroxy-1-trifluoromethylethyl)biphenyl-3-yloxymethyl]phenyl}methanol;
13-{2-Hydroxymethyl-4-[6-methyl-2'-propyl-4'-(2,2,2-trifluoro-1-hydroxy-1-trifluoromethylethyl)biphenyl-3-ylmethoxy]phenyl}methanol;
14-(2-Hydroxymethyl-4-{[6-methyl-2'-propyl-4'-(2,2,2-trifluoro-1-hydroxy-1-trifluoromethylethyl)biphenyl-3-ylamino]methyl}phenyl)methanol;
15-[2-Hydroxymethyl-4-({N-methyl[6-methyl-2'-propyl-4'-(2,2,2-trifluoro-1-hydroxy-1-trifluoromethylethyl)-biphenyl-3-yl]amino}methyl)phenyl]methanol;
16-[4-({N-Ethyl[6-methyl-2'-propyl-4'-(2,2,2-trifluoro-1-hydroxy-1-trifluoromethylethyl)biphenyl-3-yl]amino}methyl)-2-hydroxymethylphenyl]methanol;
17-[2-Hydroxymethyl-4-({[6-methyl-2'-propyl-4'-(2,2,2-trifluoro-1-hydroxy-1-trifluoromethylethyl)biphenyl-3-yl]N-propylamino}methyl)phenyl]methanol;
18-(4-{2-[6-Ethyl-4'-(1-ethyl-1-hydroxypropyl)-2'-propylbiphenyl-3-yl]ethyl}-2-hydroxymethylphenyl)methanol;
19-{4-[6-Ethyl-4'-(1-ethyl-1-hydroxypropyl)-2'-propylbiphenyl-3-ylmethoxy]-2-hydroxymethylphenyl}methanol;
20-(4-{[6-Ethyl-4'-(1-ethyl-1-hydroxypropyl)-2'-propylbiphenyl-3-ylamino]methyl}-2-hydroxymethylphenyl)methanol;
21-[4-({ [6-Ethyl-4'-(1-ethyl-1-hydroxypropyl)-2'-propylbiphenyl-3-yl]methylamino}methyl)-2-hydroxymethylphenyl]methanol;
22-[4-({Ethyl-[6-ethyl-4'-(1-ethyl-1-hydroxypropyl)-2'-propylbiphenyl-3-yl]amino}methyl)-2-hydroxymethylphenyl]methanol;
23-[4-({[6-Ethyl-4'-(1-ethyl-1-hydroxypropyl)-2'-propylbiphenyl-3-yl]propylamino}methyl)-2-hydroxymethylphenyl]methanol;
24-(4-{2-[6-Ethyl-2'-propyl-4'-(2,2,2-trifluoro-l-hydroxy-l-trifluoromethylethyl)biphenyl-3-yl]ethyl}-2-hydroxymethylphenyl)methanol;
25-{4-[6-Ethyl-2'-propyl-4'-(2,2,2-trifluoro-1-hydroxy-1-trifluoromethylethyl)biphenyl-3-yloxymethyl]-2-hydroxymethylphenyl}methanol;
26-{4-[6-Ethyl-2'-propyl-4'-(2,2,2-trifluoro-1-hydroxy-1-trifluoromethylethyl)biphenyl-3-ylmethoxy]-2-hydroxymethylphenyl}methanol;
27-(4-{[6-Ethyl-2'-propyl-4'-(2,2,2-trifluoro-1-hydroxy-1-trifluoromethylethyl)biphenyl-3-ylaminolmethyl}-2-hydroxymethylphenyl)methanol;
28-[4-({[6-Ethyl-2'-propyl-4'-(2,2,2-trifluoro-1-hydroxy-1-trifluoromethylethyl)biphenyl-3-yl]methylamino}methyl)-2-hydroxymethylphenyl]methanol;
29-4-({N-Ethyl[6-ethyl-2'-propyl-4'-(2,2,2-trifluoro-1-hydroxy-1-trifluoromethylethyl)biphenyl-3-yl]amino}methyl)-2-hydroxymethylphenyl]methanol;
30-[4-({[6-Ethyl-2'-propyl-4'-(2,2,2-trifluoro-l-hydroxy-1-trifluoromethylethyl)biphenyl-3-yl]-N-propylamino}methyl)-2-hydroxymethylphenyl]methanol;
31-(4-{[4'-(1-Ethyl-l-hydroxypropyl)-6,2'-dimethylbiphenyl-3-ylamino]methyl}-2-hydroxymethylphenyl)methanol.

2. Compounds according to Claim 1, as medicinal products.

3. Use of one or more compounds according to Claim 1, for the manufacture of a pharmaceutical composition for treating:
1- dermatological complaints associated with a differentiation or proliferation disorder of keratinocytes or sebocytes;
2- keratinization disorders;
3- dermatological complaints associated with a keratinization disorder with an inflammatory and/or immunoallergic component;
4- inflammatory complaints not showing a keratinization disorder;
5- dermal or epidermal proliferations;
6- dermatological disorders such as bullous dermatoses and collagen diseases;
7- ageing of the skin, whether photo-induced or chronological ageing, or for reducing actinic keratoses and pigmentations, or any cutaneous pathologies associated with chronological or actinic ageing;
8- cicatrization disorders and stretch marks;
9- sebaceous function disorders such as acneic hyperseborrhoea or simple seborrhoea or alternatively seborrhoeic eczema;
10- dermatological complaints with an immunological component.

4. Use according to Claim 3, **characterized in that** the dermatological complaints associated with a differentiation or proliferation disorder of keratinocytes or sebocytes relate to common acne, comedones, polymorphonuclear leukocytes, acne rosacea, nodulocystic acne, acne conglobata, senile acne and secondary acnes such as solar acne, acne medicamentosa or occupational acne.

5. Use according to Claim 3, **characterized in that** the keratinization disorders relate to ichthyosis, ichthyosiform states, Darier's disease, palmoplantar keratoderma, leukoplakia and leukoplakiform conditions, and cutaneous or mucous (buccal) lichen.

6. Use according to Claim 3, **characterized in that** the dermatological complaints associated with a keratinization disorder with an inflammatory and/or immunoallergic component concern all forms of psoriasis, whether cutaneous, mucous or ungual psoriasis, psoriatic rheumatism, and cutaneous atopy, such as eczema or respiratory atopy or gingival hypertrophy.

7. Use according to Claim 3, **characterized in that** the dermal or epidermal proliferations may be benign or malignant, of non-viral origin or of viral origin, such as common warts, flat warts and verruciform epidermodysplasia, and oral or florid papillomatoses, and **in that** the proliferations may be induced by ultraviolet radiation, especially in the case of basocellular and spinocellular epithelioma.

8. Pharmaceutical composition, **characterized in that** it comprises, in a pharmaceutically acceptable support, at least one of the compounds as defined in Claim 1.

9. Composition according to Claim 8, **characterized in that** the concentration of compound(s) according to Claim 1 is between 0.001% and 5% by weight relative to the total weight of the composition.

10. Cosmetic composition, **characterized in that** it comprises, in a cosmetically acceptable support, at least one of the compounds as defined in Claim 1.

11. Composition according to Claim 10, **characterized in that** the concentration of compound(s) is between 0.001% and 3% by weight relative to the total weight of the composition.

12. Cosmetic use of a cosmetic composition as defined in either of Claims 10 and 11, for body or hair hygiene.

13. Cosmetic use of a cosmetic composition as defined in either of Claims 10 and 11, for preventing and/or treating photo-induced or chronological ageing of the skin.

## Patentansprüche

1. Verbindungen, **dadurch gekennzeichnet, dass** sie als einzelne Verbindungen oder als Gemische aus der Gruppe ausgewählt sind, die besteht aus:
1-{5-[4'-(1-Ethyl-1-hydroxypropyl)-6-methyl-2'-propylbiphenyl-3-yloxymethyl]-2-hydroxymethylphenyl}-methanol;
2-{5-[6,2'-Diethyl-4'-(1-ethyl-1-hydroxypropyl)-biphenyl-3-yloxymethyl]-2-hydroxymethylphenyl}-methanol;
3-{4-[6-Ethyl-4'-(1-ethyl-1-hydroxypropyl)-2'-propylbiphenyl-3-yloxymethyl]-2-hydroxymethylphenyl}-methanol;
4-{4-[6-Ethyl-4'-(1-ethyl-1-hydroxypropyl)-2'-isopropylbiphenyl-3-yloxymethyl]-2-hydroxymethylphenyl}-methanol;
5-(4-{2-[4'-(1-Ethyl-1-hydroxypropyl)-6-methyl-2'-propylbiphenyl-3-yl]-ethyl}-2-hydroxymethylphenyl)-methanol;
6-{4-[4'-(1-Ethyl-1-hydroxypropyl)-6-methyl-2'-propylbiphenyl-3-ylmethoxy]-2-hydroxymethylphenyl}-methanol;
7-(4-{[4'-(1-Ethyl-1-hydroxypropyl)-6-methyl-2'-propylbiphenyl-3-ylamino]-methyl}-2-hydroxymethylphenyl)-methanol;
8-[4-({[4'-(1-Ethyl-1-hydroxypropyl)-6-methyl-2'-propylbiphenyl-3-yl]-methylamino}-methyl)-2-hydroxymethylphenyl]-methanol;
9-[4-({Ethyl-[4'-(1-ethyl-1-hydroxypropyl)-6-methyl-2'-propylbiphenyl-3-yl]-amino}-methyl)-2-hydroxymethylphenyl]-methanol;
10-[4-({[4'-(1-Ethyl-1-hydroxypropyl)-6-methyl-2'-propylbiphenyl-3-yl]-propylamino}-methyl)-2-hydroxymethylphenyl]-methanol;
11-(2-Hydroxymethyl-4-{2-[6-methyl-2'-propyl-4'-(2,2,2-trifluor-1-hydroxy-1-trifluormethyl-ethyl)-biphenyl-3-yl]-ethyl}-phenyl)-methanol;
12-{2-Hydroxymethyl-4-[6-methyl-2'-propyl-4'-(2, 2,2-trifluor-1-hydroxy-1 trifluormethyl-ethyl)- biphenyl-3-yloxymethyl]-phenyl}-methanol;
13-{2-Hydroxymethyl-4-[6-methyl-2'-propyl-4'-(2, 2,2-trifluor-1-hydroxy-1 -trinuormethyl-ethyl)-biphenyl-3-ylmcthoxy]-phenyl}-methanol;
14-(2-Hydroxymethyl-4-{[6-methyl-2'-propyl-4'-(2,2,2-trifluor-1-hydroxy-1-trifluormethyl-ethyl)-biphenyl-3-ylamino]-methyl}-phenyl)-methanol;
15-[2-Hydroxymethyl-4-({N-methyl[6-methyl-2'-propyl-4'-(2,2,2-trifluor-1-hydroxy-1-trifluormethyl-ethyl)-biphenyl-3-yl]-amino}-methyl)-phenyl]-methanol;
16-[4-({N-Ethyl-[6-methyl-2'-propyl-4'-(2,2,2-trifluor-1-hydroxy-1-trifluormethyl-ethyl)-biphenyl-3-yl]-amino}-methyl)-2-hydroxymethylphenyl]-methanol;
17-[2-Hydroxymethyl-4-({[6-methyl-2'-propyl-4'-(2,2,2-trifluor-1-hydroxy-1-trifluormethyl-ethyl)-biphenyl-3-yl]-N-propylamino}-methyl)-phenyl]-methanol;
18-(4-{2-[6-Ethyl-4'-(1-ethyl-1-hydroxypropyl)-2'-propylbiphenyl-3-yl]-ethyl}-2-hydroxymethylphenyl)-methanol;
19-{4-[6-Ethyl-4'-(1-ethyl-1-hydroxypropyl)-2'-propylbiphenyl-3-ylmethoxy]-2-hydroxymethylphenyl}-methanol;
20-(4-{[6-Ethyl-4'-(1-ethyl-1-hydroxypropyl)-2'-propylbiphenyl-3-ylamino]-methyl}-2-hydroxymethylphenyl)-methanol;
21-[4-({[6-Ethyl-4'-(1-ethyl-1-hydroxypropyl)-2'-propylbiphenyl-3-yl]-methylamino}-methyl)-2-hydroxymethylphenyl]-methanol;
22-[4-({Ethyl-[6-ethyl-4'-(1-ethyl-1-hydroxypropyl)-2'-propylbiphenyl-3-yl]-amino}-methyl)-2-hydroxymethylphenyl]-methanol;
23-[4-({[6-Ethyl-4'-(1-ethyl-1-hydroxypropyl)-2'-propylbiphenyl-3-yl]-propylamino}-methyl)-2-hydroxymethylphenyl]-methanol;
24-(4-{2-[6-Ethyl-2'-propyl-4'-(2,2,2-trifluor-1-hydroxy-1-trifluormethyl-ethyl)-biphenyl-3-yl]-ethyl}-2-hydroxymethylphenyl)-methanol;
25-{4-[6-Ethyl-2'-propyl-4'-(2,2,2-trifluor-1-hydroxy-1-trifluormethyl-ethyl)-biphenyl-3-yloxymethyl]-2-hydroxymethylphenyl}-methanol;
26-{4-[6-Ethyl-2'-propyl-4'-(2,2,2-trifluor-1-hydroxy-1-trifluormethyl-ethyl)-biphenyl-3-ylmethoxy]-2-hydroxymethylphenyl}-methanol;
27-(4-{[6-Ethyl-2'-propyl-4'-(2,2,2-trifluor-1-hydroxy-1-trifluormethyl-ethyl)-biphenyl-3-ylamino]-methyl}-2-hydroxymethylphenyl)-methanol;
28-[4-({[6-Ethyl-2'-propyl-4'-(2,2,2-trifluor-1-hydroxy-1-trifluormethyl-ethyl)-biphenyl-3-yl]-methylamino}-methyl)-2-hydroxymethylphenyl]-methanol;
29-[4-({N-Ethyl-[6-ethyl-2'-propyl-4'-(2,2,2-trifluor-1-hydroxy-1-trifluormethyl-ethyl)-biphenyl-3-yl]-amino}-methyl)-2-hydroxymethylphenyl]-methanol;
30-[4-({[6-Ethyl-2'-propyl-4'-(2,2,2-trifluor-1-hydroxy-1-trifluormethyl-ethyl)-biphenyl-3-yl]-N-propyl-amino}-methyl)-2-hydroxymethylphenyl]-methanol;
31-(4-{[4'-(1-Ethyl-1-hydroxypropyl)-6,2'-dimethylbiphenyl-3-ylamino]-methyl}-2-hydroxymethylphenyl)-methanol.

2. Verbindungen nach Anspruch 1 als Arzneimittel.

3. Verwendung einer oder mehrerer Verbindungen nach Anspruch 1 zur Herstellung einer pharmazeutischen Zusammensetzung, die vorgesehen ist zur Behandlung von:
1- dermatologischen Erkrankungen, die mit einer Störung der Differenzierung oder der Proliferation der Keratinocyten oder der Sebocyten in Zusammenhang stehen;
2- Störungen der Keratinisierung;
3- dermatologischen Erkrankungen, die mit einer Störung der Keratinisierung mit einer entzündlichen und/oder immunoallergischen Komponente in Zusammenhang stehen;
4- entzündlichen Erkrankungen, die nicht mit einer Störung der Keratinisierung einhergehen;
5- Proliferationen der Dermis oder der Epidermis;
6- dermatologischen Störungen, wie bullösen Dermatosen und Erkrankungen des Collagens;
7- der Alterung der Haut unabhängig davon, ob sie lichtinduziert oder altersbedingt ist, oder zur Verringerung von aktinischen Pigmentationen und Keratosen oder von allen mit altersbedingter oder aktinischer Alterung in Zusammenhang stehenden pathologischen Zuständen;
8- Störungen der Narbenbildung und von Schwangerschaftsstreifen;
9- Störungen der Talgdrüsenfunktion, wie hyperseborrhoischer Akne oder einfacher Seborrhoe oder auch von seborrhoischem Ekzem;
10- dermatologischen Erkrankungen mit immunologischer Komponente.

4. Verwendung nach Anspruch 3, **dadurch gekennzeichnet, dass** die dermatologischen Erkrankungen, die mit einer Störung der Differenzierung oder der Proliferation der Keratinocyten oder der Sebocyten in Zusammenhang stehen, sich beziehen auf *Acne vulgaris,* Komedonenaknen, polymorphe Aknen, *Acne rosacea,* nodulocystische Aknen, *Acne conglobata,* senile Aknen, sekundäre Aknen wie Sonnenakne, Arzneimittelakne oder Berufsakne.

5. Verwendung nach Anspruch 3, **dadurch gekennzeichnet, dass** sich die Störungen der Keratinisierung beziehen auf Ichthyosen, ichthyosiforme Zustände, die Darier-Krankheit, palmoplantare Keratodermien, Leukoplasien und leukoplasiforme Zustände, Lichen der Haut oder der Schleimhäute (bukkal).

6. Verwendung nach Anspruch 3, **dadurch gekennzeichnet, dass** die dermatologischen Erkrankungen, die mit einer Störung der Keratinisierung mit einer entzündlichen Komponente und/oder immunoallergischen Komponente in Zusammenhang stehen, sich beziehen auf alle Formen der Psoriasis, d.h. auf Psoriasis der Haut, der Schleimhäute oder der Nägel oder psoriatisches Rheuma, sowie auf Hautatopien, wie Ekzeme, oder auf respiratorische Atopie oder gingivale Hypertrophie.

7. Verwendung nach Anspruch 3, **dadurch gekennzeichnet, dass** es sich bei den Proliferationen der Dermis oder der Epidermis um benigne oder maligne Proliferationen nichtviralen Ursprungs oder viralen Ursprungs handelt, wie zum Beispiel um gewöhnliche Warzen, Flachwarzen und warzenartige Epidermodysplasien, orale oder floride Papillomatosen, und dass die Proliferationen durch Ultraviolettstrahlung induziert sein können, insbesondere im Fall von Basaliomen und spinocellulären Epitheliomen.

8. Pharmazeutische Zusammensetzung, **dadurch gekennzeichnet, dass** sie in einem pharmazeutisch akzeptablen Träger mindestens eine der Verbindungen enthält, wie sie in Anspruch 1 definiert sind.

9. Zusammensetzung nach Anspruch 8, **dadurch gekennzeichnet, dass** die Konzentration an Verbindung(en) nach Anspruch 1 im Bereich von 0,01 bis 5 Gew.-% liegt, bezogen auf das Gesamtgewicht der Zusammensetzung.

10. Kosmetische Zusammensetzung, **dadurch gekennzeichnet, dass** sie in einem kosmetisch akzeptablen Träger mindestens eine der Verbindungen enthält, wie sie in Anspruch 1 definiert sind.

11. Zusammensetzung nach Anspruch 10, **dadurch gekennzeichnet, dass** die Konzentration an Verbindung(en) im Bereich von 0,001 bis 3 Gew.-% liegt, bezogen auf das Gesamtgewicht der Zusammensetzung.

12. Kosmetische Verwendung einer kosmetischen Zusammensetzung, wie sie in Anspruch 10 oder 11 definiert ist, zur Körperhygiene oder Haarhygiene.

13. Kosmetische Verwendung einer kosmetischen Zusammensetzung, wie sie in Anspruch 10 oder 11 definiert ist, zur Vorbeugung und/oder Behandlung der lichtinduzierten oder altersbedingten Alterung der Haut.
